# EUROPEAN PATENT SPECIFICATION

(11) **EP 3 775 053 B1**
(45) Date of publication and mention of the grant of the patent: **06.09.2023**
(21) Application number: 19781458.5
(22) Date of filing: 04.04.2019
(51) Int. Cl.: A61K 9/51, A61K 38/49, A61K 47/69, A61P 7/02, A61P 9/10, A61P 17/02

(54) **FIBRIN-SPECIFIC MATERIALS AND METHODS OF USING THEREOF**
FIBRINSPEZIFISCHE MATERIALIEN UND VERFAHREN ZU DEREN VERWENDUNG
MATÉRIAUX SPÉCIFIQUES À LA FIBRINE ET PROCÉDÉS D'UTILISATION CORRESPONDANTS

(30) Priority: 04.04.2018 US 201862652654 P; 10.05.2018 US 201862669616 P
(43) Date of publication of application: 17.02.2021
(73) Proprietor: North Carolina State University, Raleigh, North Carolina 27606 (US)
(72) Inventor: BROWN, Ashley, Raleigh, North Carolina 27606 (US); CHENG, Ke, Raleigh, North Carolina 27606 (US); MIHALKO, Emily, Raleigh, North Carolina 27606 (US); SPROUL, Erin, Raleigh, North Carolina 27606 (US)
(74) Representative: Creek, Isobel Clare
(86) International application number: PCT/US2019/025839
(87) International publication number: WO 2019/195580

(56) References cited:
- WO-A1-2015/070233
- WO-A2-2012/074588
- US-A1- 2014 200 262
- US-A1- 2016 346 327
- BROWN et al.: "Ultrasoft Microgels Displaying Emergent, Platelet-Like, Behaviors", Nature Materials, vol. 13, no. 12, December 2014 (2014-12), pages 1-12, XP055628598, DOI: 10.1038/nmat4066
- WELSCH et al.: "Enhancing Clot Properties Through Fibrin-Specific Self-Cross-Linked PEG Side-Chain Microgels", Colloids and Surfaces B: Biointerfaces, vol. 166, 1 June 2018 (2018-06-01), pages 89-97, XP085382184,

## Description

### TECHNICAL FIELD

This application relates generally to fibrin-specific nanogels that can be used for drug delivery, to promote wound healing, to treat thrombotic events, and to treat coagulative disorders.

### BACKGROUND

Fibrin (also called Factor Ia) is a fibrous, non-globular protein involved in the clotting of blood. Fibrin is formed by the action of the protease thrombin on fibrinogen which causes it to polymerize. Polymerized fibrin, together with platelets, forms a hemostatic plug or clot over a wound site.

Owing to its role in the coagulation cascade, fibrin can be involved in many disease processes within the body. For example, excessive generation of fibrin due to activation of the coagulation cascade leads to thrombosis, the blockage of a vessel by an agglutination of red blood cells, platelets, polymerized fibrin and other components. Ineffective generation or premature lysis of fibrin can increase the likelihood of a hemorrhage. Likewise, fibrin is important for clotting and wound healing. Thus, materials which target, interact with, and/or modulate fibrin and associated pathways offer promise in a wide number of biomedical applications.

For example, particles which target, interact with, and/or modulate fibrin can aid in wound treatment. Platelets mediate hemostasis by aggregating and binding to fibrin to promote clotting. Over time, platelets contract the fibrin network to induce clot retraction, which contributes to wound healing outcomes by increasing clot stability and improving blood flow to ischemic tissue. After a blood vessel is damaged, platelets aggregate around the wound. The platelets have thrombin receptors which bind serum thrombin. Thrombin catalyzes the conversion of soluble fibrinogen into fibrin, which is polymerized primarily through noncovalent interactions. Finally, factor XIII covalently crosslinks the polymerized fibrin to produce a clot. The inability of a wound to heal quickly is a key concern in managing major injuries, especially in patients with impaired coagulation abilities, such as hemophiliacs, diabetics, and sufferers of Von Willebrand disease.

Due to the fundamental role of platelets in hemostasis, there has been great interest in developing synthetic platelets or platelet-like particles ("PLPs") that interact with the native coagulation cascade to promote clotting and treat traumatic injury. A number of platelet-mimetic materials have been developed in recent years to reproduce the hemostatic properties of platelets; these efforts have recently been reviewed. Overall, approaches to synthetic platelet design focus on replicating various platelet functions. One such example mimics platelet morphology to recreate platelet margination. Other synthetic platelet designs incorporate a nanoparticle decorated with binding domains to mimic platelet binding to coagulation components. Some notable examples include RGD-PLL-PLGA particles, albumin microparticles coated with fibrinogen, and liposomal particles conjugated with GPIb. Recent studies have also demonstrated that synthetic platelets encompassing collagen-binding and vWF-binding motifs improve binding to wound sites under flow conditions. Injectable, shear-thinning composite hydrogels containing gelatin and silicate nanoplatelets have also been described for use as an embolic agent for endovascular embolization procedures.

The remains a need for improved platelet-like particles with improved clinical utility and ease of administration

Particles which target, interact with, and/or modulate fibrin also have promise for the treatment of diseases or disorders associated with thrombosis. By way of example, approximately 790,000 new or recurrent cases of myocardial infarct (MI) occur each year in the United States. MI typically occurs as the result of plaque rupture and thrombus formation, which occludes the coronary artery and leads to ischemic damage to the heart tissue. MI is also accompanied by excessive fibrin deposition within the extracellular matrix. The coagulation protein fibrin is a major component of the resulting thrombus, and thus represents a prime target for therapeutic intervention. In treating MI, reestablishing blood flow to the ischemic tissue is crucial to prevent death. However, reperfusion itself can lead to cardiomyocyte death and scar tissue formation that impairs cardiac function. No current treatment strategy for MI simultaneously addresses the need to both reestablish blood flow and protect the heart from ischemic reperfusion (I/R) injury.

Reperfusion therapy normally depends on vessel recanalization by catheters by percutaneous coronary intervention (PCI). However, these procedures can only be done in relatively large vessels, leaving the occlusion of small vessels untreatable. Furthermore, in rural and emergency medicine settings, cath labs are not readily available. In such settings, anticoagulants and fibrinolytic compounds, such as tissue plasminogen activator (tPA), are often used to treat MI in both large and tiny vessels. However, intravenous administration of fibrinolytic compounds comes with the inherent risk of severe hemorrhage due to off-target binding at non-disease sites. Therefore, utilizing a targeted approach for the delivery of fibrinolytic compounds to sites of MI could minimize potential off-target bleeding complications and enable fast and effective dissolution of MI-associated thrombi. In our therapeutic nanogel design, in order to quickly reestablish blood flow, nanogels will first release a fibrinolytic protein, tissue plasminogen activator (tPA), to lyse the immediate MI occlusion.

Disseminated intravascular coagulation (DIC) is a pathological process that causes abnormal widespread clotting cascade activation, forming blood clots in small blood vessels throughout the body. While this over-activation of clotting leads to tissue damage and multi-organ failure, DIC also contributes to severe bleeding as clotting factors are consumed in other areas of the body. DIC occurs in conditions such as solid tumors, blood cancers, severe trauma, and infections. Current treatment of DIC mainly involves treating the underlying conditions, but platelet or plasma transfusions are administered in cases of severe bleeding. Additionally, anticoagulants, such as heparin, may be used to treat thrombosis in DIC.

Although reperfusion injury and subsequent cardiac scarring is recognized as a major clinical problem, no effective therapies currently exist to effectively treat this condition. In fibrotic conditions, activities such as Rho GTPase signaling, actin cytoskeleton engagement, mechanical activation of transforming growth factor beta, and fibroblast activation and myofibroblast differentiation become over-activated and contribute to the onset and progression of fibrotic responses. Additionally, cell contractility has been linked to such fibrotic responses in multiple organ systems. Specifically, Rho-ROCK signaling, which plays an important role in how cells sense and generate a response to their environment through actin-cytoskeleton stabilization, has been shown to increase in cardiac fibrosis. This leads to overexpression of fibrotic-related genes, in particular, connective tissue growth factor (CTGF). Therefore, inhibition of the ROCK pathway represents a promising therapeutic target for mitigating fibrotic responses. Indeed, it has recently been demonstrated that the small molecule ROCK inhibitor Y-27632 is capable of abrogating pathological processes in fibrosis, such as promotion of myofibroblastic differentiation. However, because of its central role in mediating the organization of the actin cytoskeleton, ROCK plays a role in a wide range of fundamental cellular activities including adhesion, migration, proliferation and gene expression. Therefore, it is not desirable to inhibit ROCK systemically, as this could have deleterious off-target effects.

There remains a need for improved methods to quickly reestablish blood flow and inhibit fibrosis following a thrombotic event such as myocardial infarction, stroke, limb ischemia, pulmonary embolism, deep vein thrombosis, or hepatic artery thrombosis. There remains a need for additional methods of treating MI that do not require percutaneous coronary intervention and other elaborate surgical procedures. There remains a need for methods of selectively delivering fibrinolytic agents to occlusions without target non-affected tissues

### SUMMARY

Provided herein are fibrin-specific nanogels. The fibrin-specific nanogels can comprise a fibrin binding moiety conjugated to a polymeric matrix (e.g., a crosslinked polyacrylamide matrix). By varying the morphology of fibrin-specific nanogels (e.g., by varying the crosslinker density and/or the three-dimensional structure of the fibrin-specific nanogels), fibrin-specific materials which mimic the biophysical characteristics of platelets can be formed.

In some embodiments, the fibrin-specific nanogels can be hollow particles comprising a shell formed from a polymeric matrix (e.g., a crosslinked polyacrylamide matrix) enveloping a hollow core. A fibrin binding moiety can be conjugated to the polymeric matrix (e.g., the crosslinked polyacrylamide matrix) forming the shell.

In other embodiments, the fibrin-specific nanogels can be core-shell particles comprising a core comprising a first crosslinked polymer network; and a shell comprising a second crosslinked polymer network at least partially surrounding the core. In some embodiments, the shell can completely envelope the core. The first crosslinked polymer network, the second crosslinked polymer network, or a combination thereof can comprise a crosslinked polyacrylamide matrix. In some embodiments, the crosslinking density of the core can be different than the crosslinking density of the shell (e.g., the crosslinking density of the core can be greater than the crosslinking density of the shell). A fibrin binding moiety can be conjugated to the polymeric matrix (e.g., the crosslinked polyacrylamide matrix) forming the shell.

The resulting materials can be used in a variety of biomedical applications (e.g., for drug delivery, to promote wound healing, to treat thrombotic events, and to treat coagulative disorders).

In certain examples, provided herein are nanogel platelet-like particles ("PLPs") useful for promoting wound healing and drug delivery. The PLPs can be hollow and exhibit improved deformability relative to other synthetic platelet mimics. The nanogels can include a variety of therapeutic agents, including proteins and small molecule drugs like analgesics, antibiotics, anti-inflammatories, immunosuppressants, among others. The nanogel systems can promote wound healing, and can be directly applied to a wound site, or delivered systemically.

In certain examples, provided herein are fibrinolytic nanogels useful for a variety of thrombotic and coagulative disorders. The nanogels include a fibrin binding moiety and tissue plasminogen activator, and optionally additional therapeutic agents. The nanogel systems quickly reestablish blood flow and inhibit cardiac fibrosis following reperfusion injury. Therefore, to prevent cardiac fibrosis following myocardial reperfusion injury, the therapeutic nanogels described herein can release a fibrotic inhibitor. The nanogels mitigate bleeding complications associated with systemic delivery of a fibrinolytic protein, as well as protect against the deleterious off-target effects of systemic delivery of a cell contractility inhibitor.

The details of one or more embodiments are set forth in the descriptions below. Other features, objects, and advantages will be apparent from the description and from the claims.

### DESCRIPTION OF DRAWINGS

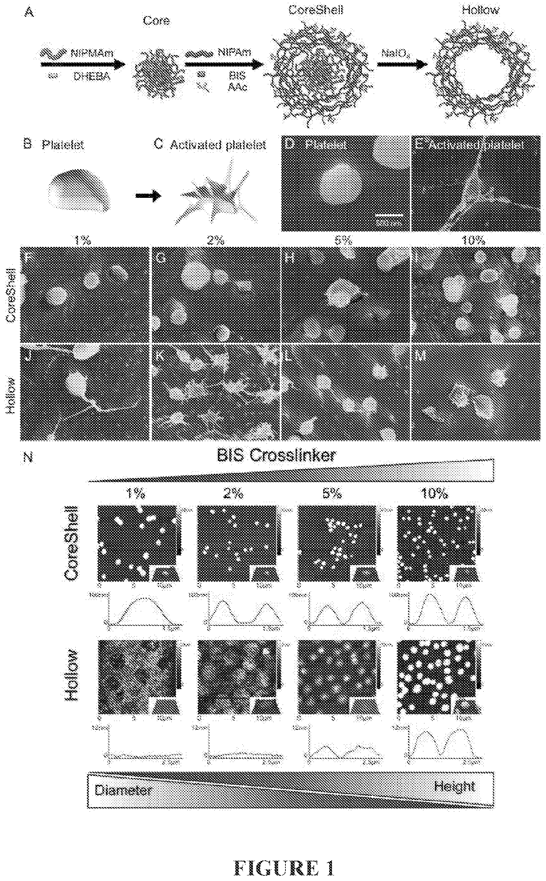

Figure 1 illustrates that the hollow microgel structure imparts morphology similar to native platelets. Panel A shows a schematic of hollow microgel synthesis following core degradation resulting in a deformable outer shell. Native circulating platelets display an ovoid morphology (panel B) that upon activation, forms spindle-like projections illustrated in panel C and imaged by CryoSEM imaging in panel D and panel E. CryoSEM imaging of microgel morphology was performed with a JEOL 7600F at 50000X magnification. At least 5 images at 50000X per condition were collected and representative images are shown. CoreShell microgels (panels F-I) illustrates a morphology similar to native resting platelets and corresponding hollow microgels (panels J-M) resemble activated platelets with spindle-like projections. Atomic Force Microscopy (AFM) analysis (panel N) demonstrated that hollow microgels display high deformability and spread more extensively on surfaces than CS microgels. Diameter and height traces were generated with Asylum AFM software for at least 30 microgels per condition from at least 3 different images. Representative images and height traces are shown.

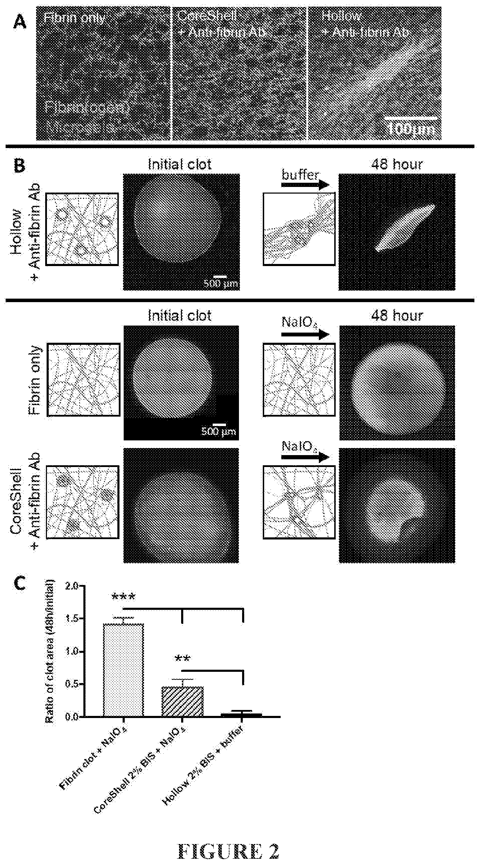

Figure 2 illustrates that particle deformability is important for clot retraction. As shown in panel A, confocal microscopy demonstrated that when coupled to a fibrin-binding antibody, 2% BIS crosslinked hollow microgels increased fibrin network density, while intact CoreShell microgels did not. A minimum of 6 clots were analyzed per group and representative images are presented. Fibrin clot collapse can be seen in a bulk clot collapse where hollow particles induce contraction (panel B). Bulk clot collapse of CoreShell microgels conjugated to a fibrin-binding antibody, polymerized into a fibrin clot and then subjected to core degradation via 50 mM NaIO₄ was also analyzed. Upon core degradation, gross fibrin clot collapse was observed, indicating that this design facilitated "triggerable" clot retraction. Clot area was measured at 48 hr and normalized to initial clot area (panel C) for at least n=3 clots per group; representative images are presented in panel B. Significance was determined with a one-way ANOVA with a post hoc Tukey's multiple comparisons test. ** p < 0.01; *** p < 0.001.

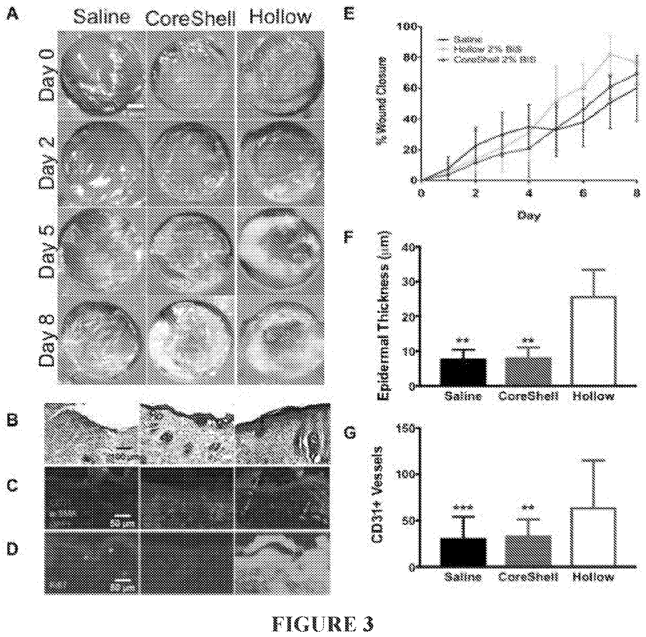

Figure 3 illustrates the ability of hollow PLPs to enhance wound healing outcomes *in vivo.* Wound healing following topical application of saline, CS, or hollow PLPs was analyzed in a murine full thickness dermal injury model (n=5/group). Representative images of wounds at 0, 2, 5, and 8 days post-injury are shown in panel A. Histology of wound cross-sections at day 8 with MSB staining (panel B) revealed a thicker epidermis in response to hollow PLP treatment compared to saline or CS PLP groups. IHC of wound cross-sections also showed an increase in CD31⁺ vessels (panel C), indicating the presence of endothelial cells forming new blood vessels, or angiogenesis, which is a critical step in wound healing. Additionally, IHC showed an increase in proliferation marker Ki67 (panel D). Percent wound closure is plotted in panel E. Wound area was measured each day over an 8-day period and normalized to the wound's corresponding initial area. N=5/group. Mean and SD are presented at each time point. Epidermal thickness was quantified (panel F) from MSB images at Day 8 by measuring 5+ areas per image for n=4 images. CD31 positively labeled tissue was quantified (panel G) by measuring total number of CD31+ vessels per 40X image for at least 15 histological samples for each treatment condition. Statistical analysis was performed using a one-way ANOVA with a post hoc Tukey's multiple comparisons test for epidermal thickness measurements and a Kruskal-Wallis with Dunn's multiple comparisons test for CD31+ vessel counts to analyze statistical significance with significance determined as p<0.05. ** p < 0.01; ***p<0.001.

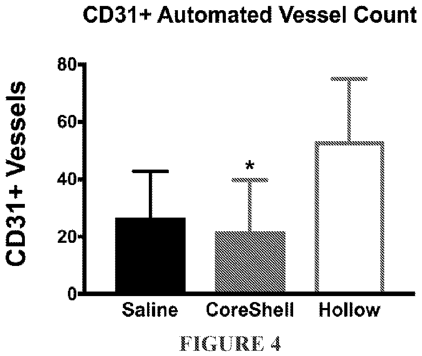

Figure 4 shows the results of an automated analysis of CD31+ vessels. Wound healing following topical application of saline, CS, or hollow PLPs was analyzed in a murine full thickness dermal injury model (n=5/group). IHC of wound cross-sections showed an increase in CD31⁺ vessels, indicating the presence of endothelial cells forming new blood vessels, or angiogenesis. CD31 positively labeled tissue was quantified by ImageJ using Particle Analysis to measure total red area (greater than 0.5 µm with a threshold 0-50) for at least 15 samples for each treatment condition per 40X image. Statistical analysis was performed using a Kruskal-Wallis with Dunn's multiple comparisons test with significance determined as p<0.05. * p < 0.05.

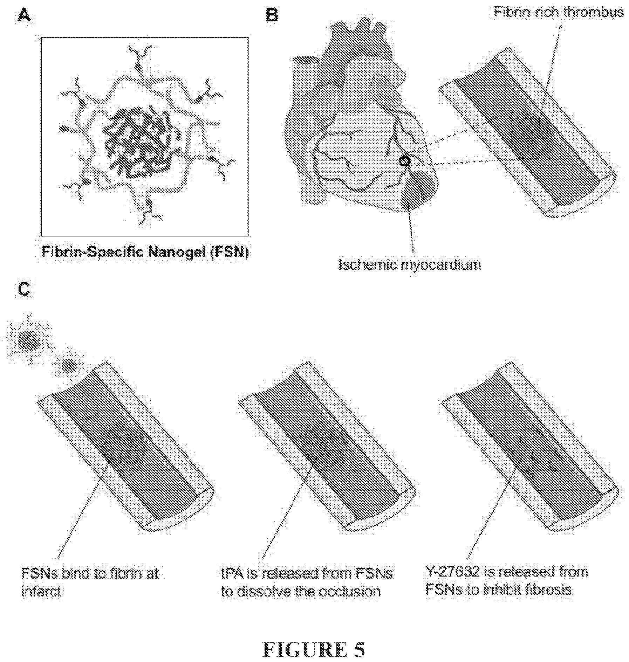

Figure 5 shows a fibrin-specific nanogel design (panel A) that, when drug-loaded, combats myocardial infarction, which occurs due to a fibrin rich thrombus blocking blood flow and creating ischemic myocardium (panel B). Drug-loaded FSNs will bind to fibrin at the infarct site, release a fibrinolytic drug to dissolve the immediate fibrin-rich occlusion, then release a small molecule cell contractility inhibitor to mitigate cardiac fibrosis due to reperfusion injury (panel C).

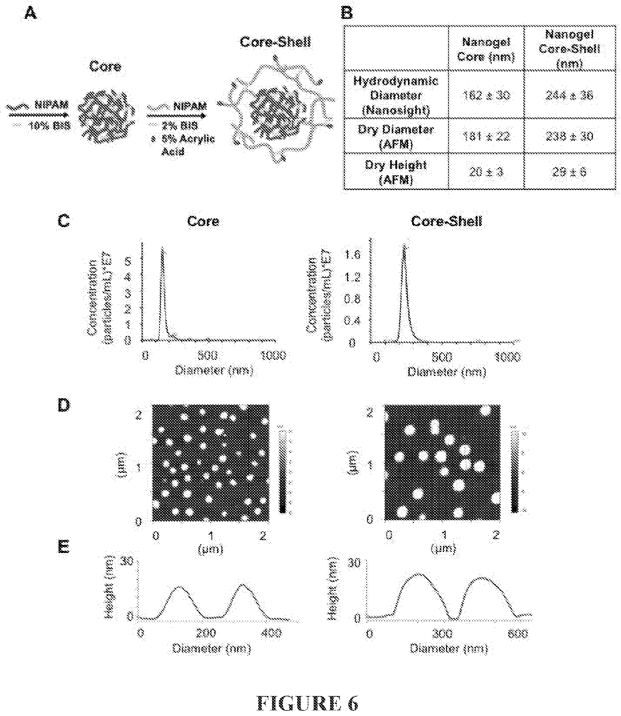

Figure 6 shows a core and core-shell nanogel (panel A) and summarizes their size characterization (panel B). Nanosight particle tracking for hydrodynamic diameter measurements (panel C) as well as AFM images on glass surface (panel D) and height traces of core and core-shell nanogels (panel E) show nanogel sizes before and after shell addition. Nanosight and AFM analysis demonstrate a significant increase in size following shell addition, indicating successful shell addition. n=≥30/group.

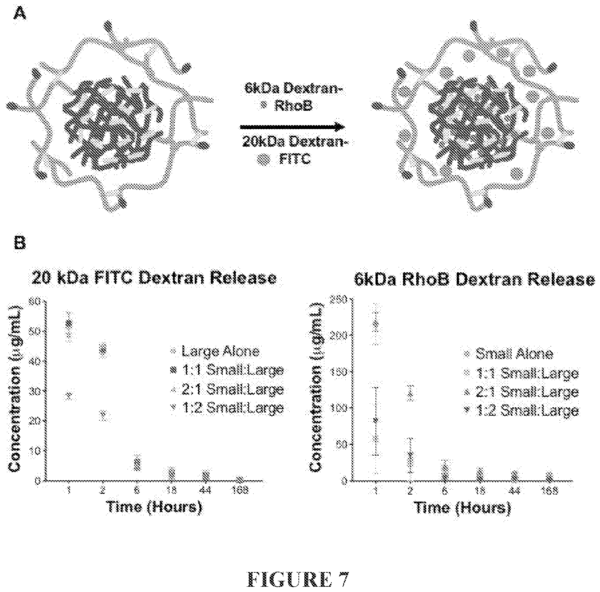

Figure 7 shows the analysis of dextran release following C/S nanogel loading of small and large fluorescently labeled dextran molecules (panel A). Release profiles of varying ratios of large (20kDa) and small (6kDa) dextran molecules over the course of 168 hours are shown in panel B. n=3/group.

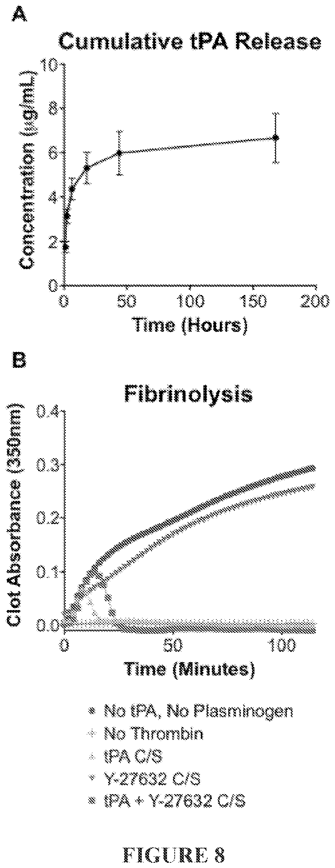

Figure 8 shows the cumulative tPA release from C/S nanogels (panel A) and the analysis of fibrinolysis in the presence of drug-loaded nanogels (panel B). n=3 triplicate experiments/group.

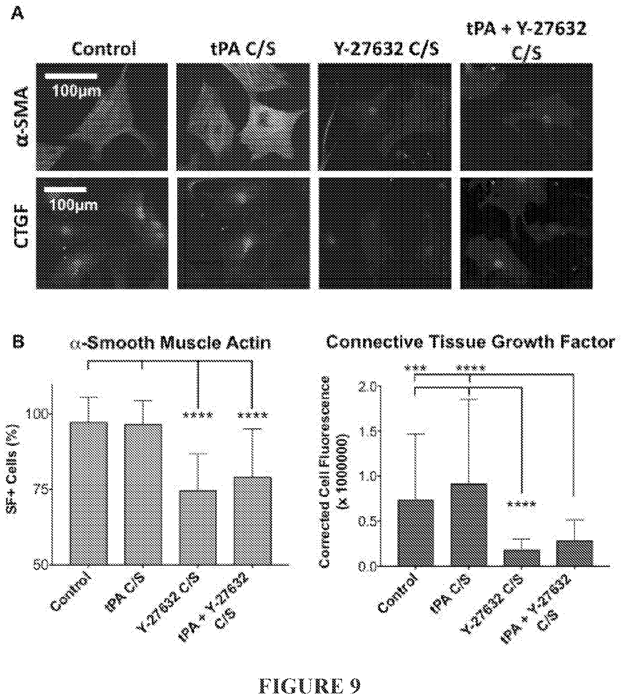

Figure 9 shows the *in vitro* analysis of fibrotic markers, α-SMA and CTGF, with decreased expression in the presence of drug-loaded C/S particles (panel A) and quantification using percent stress fiber positive cells for α-SMA and corrected total cell fluorescence for CTGF (panel B). n=3 duplicate experiments with a minimum of 20 cells/group analyzed/replicate. ***p<0.001; ****p<0.0001.

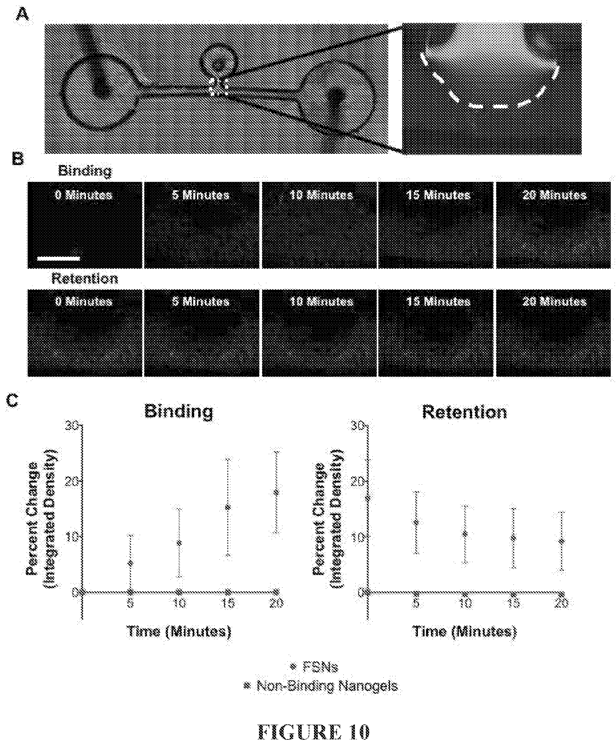

Figure 10 shows that FSNs bind to and are retained at fibrin clot boundaries *in vitro* at wall shear rates of 1sec⁻¹. PDMS molds were fabricated and a fibrin clot polymerized along the channel (panel A). Binding over the course of 20 minutes and retention during a buffer wash for 20 minutes (panel B) show accumulation at fibrin clot sites, quantified by fluorescence intensity at the clot boundary (panel C). n=3/group.

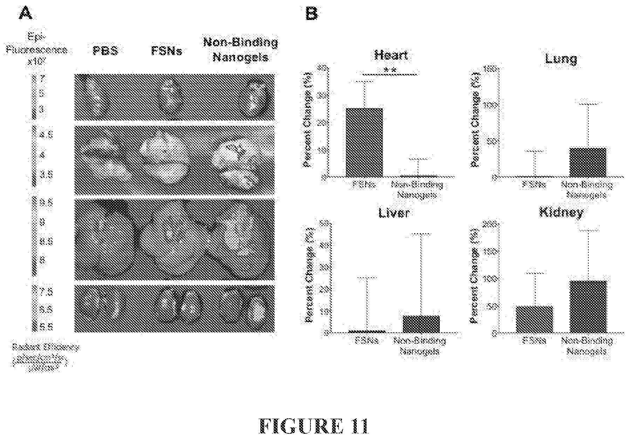

Figure 11 shows FSNs retained at sites of MI *in vivo* (panel A), quantified by the percent change in fluorescence intensity normalized to PBS treatment controls (panel B). n=5/group. **p<0.01.

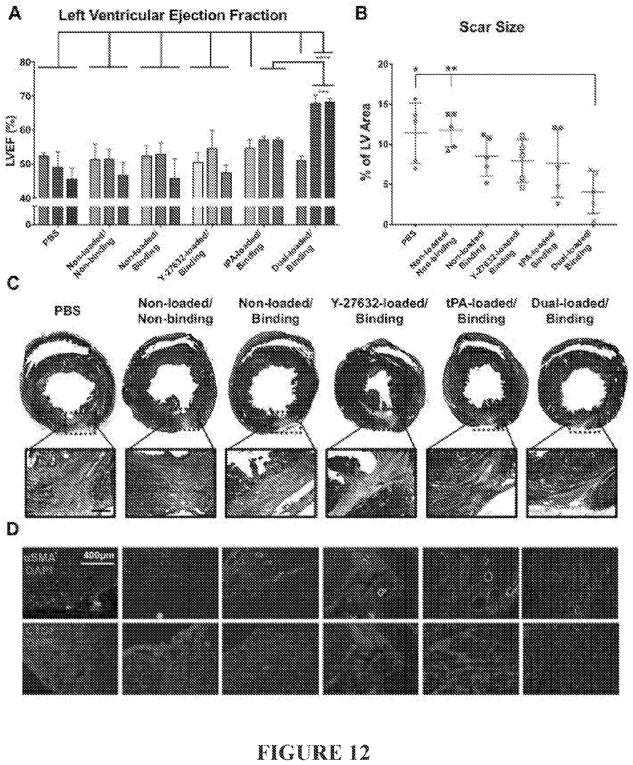

Figure 12 shows that dual-loaded FSNs significantly improve left ventricular ejection fraction 2 and 4 weeks following I/R *in vivo* (panel A). n=5/group. ***p<0.001 **** p<0.0001. At 4 weeks post-injury, dual-loaded FSNs significantly decrease scar size (panel B) quantified through Masson's trichrome staining and measuring blue collagen stain as a percent of left ventricular area (panel C). n=5/group, *p<0.05 **p<0.01. Dual-loaded FSNs decrease α-SMA (top) and CTGF (bottom) expression in vivo 4 weeks following I/R (panel D). n=5/group. Representative images are shown.

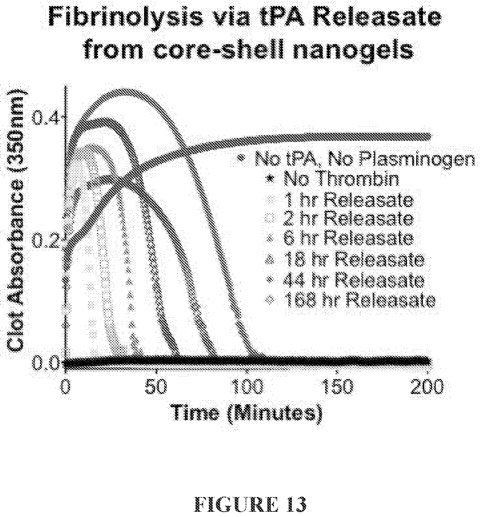

Figure 13 shows an analysis of fibrinolysis in the presence of supernatant released from tPA loaded particles. Representative fibrinolysis plot shown.

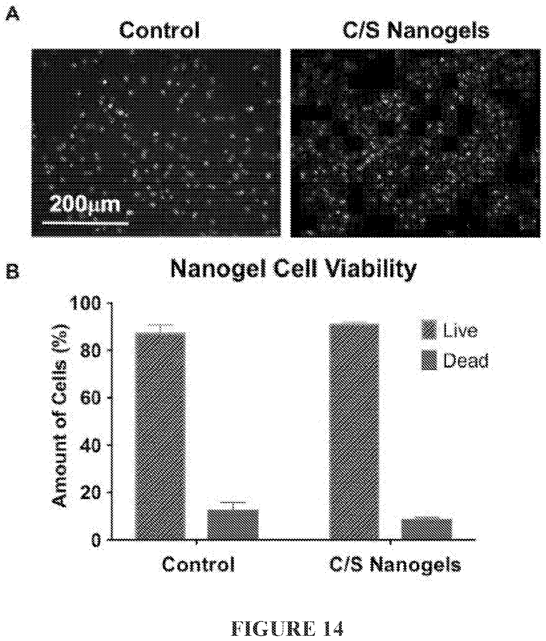

Figure 14 shows the results of a live/dead assay on neonatal rat cardiac fibroblasts in the presence or absence (control) of core-shell nanogels. n=3 triplicate experiments. Representative images shown above.

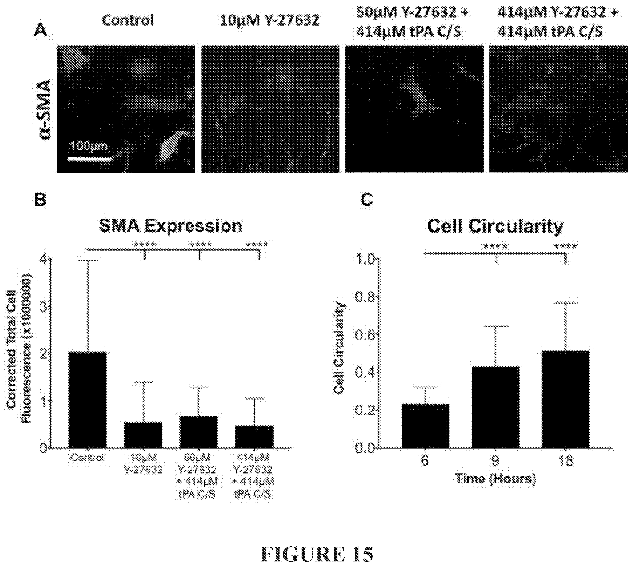

Figure 15 shows the *in vitro* analysis of decreased α-SMA expression of HDFn cells in the presence of drug-loaded C/S particles (panel A) and quantification using corrected total cell fluorescence (panel B). Increased cell circularity in the presence of Y-27632 loaded particles (C). n=3 experiments with a minimum of 20 cells analyzed per time point for each experiment. **** p<0.0001.

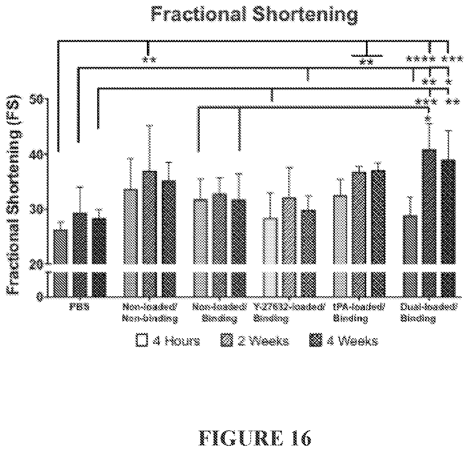

Figure 16 shows that dual-loaded FSNs significantly improve fractional shortening 2 and 4 weeks following I/R *in vivo.*

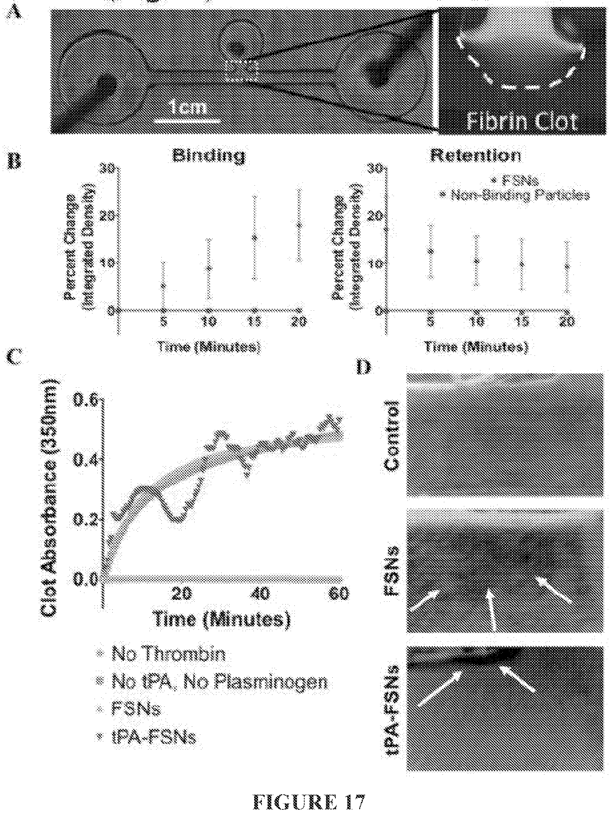

Figure 17 shows the PDMS device used to visualize FSN binding (panel A), FSN binding at fibrin clot boundaries at wall shear rates of 1sec⁻¹ (panel B, N=3/group, dynamic polymerization/fibrinolysis plots demonstrating clot lysis and destabilization in the presence of tPA loaded FSNs (panel c, N=3/group), endpoint images of fibrin clots in the presence of FSNs showing significant clotting (arrows, panel d). In the presence of tPA-FSNs, enhanced clotting is localized around the stationary clot boundary site (arrows, panel d). Images are at 4x magnification.

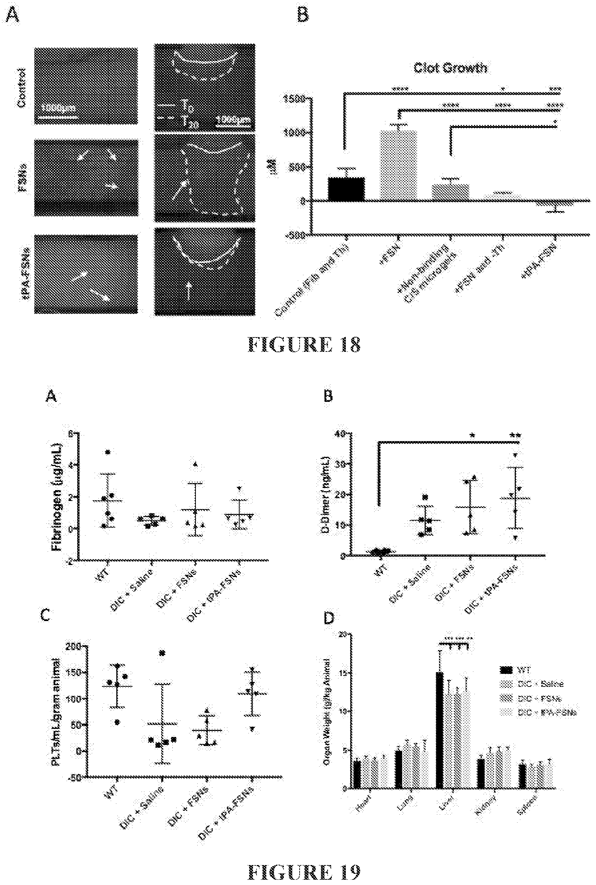

Figure 18 shows the *in vitro* clotting characteristics using a polydimethylsiloxane fluidics chamber with pre-polymerized fibrin clot at a T-intersection and flowing solutions of fibrinogen and thrombin that contain FSNs, non-binding microgels, FSNs alone without thrombin, and tPA-FSNs.
Figure 19 shows the fibrinogen (panel A), D-dimer (panel B), platelet count (panel C), and organ weights (panel D) measured in DIC-induced animals treated with either saline, FSNs, or tPA-FSNs and compared to naive wildtype animals.

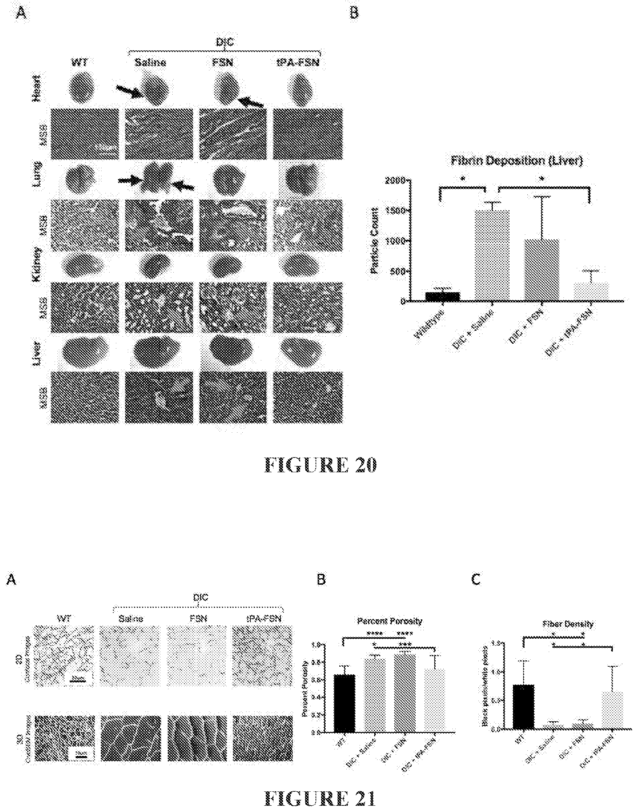

Figure 20 shows whole organ images of wildtype and DIC-induced animals treated with saline, FSNs, or tPA-FSNs. Black arrows point to dark, hemorrhagic areas in the organs. Martius scarlet blue trichrome stain for fibrin (red) is shown for histological sections and red arrows point to areas of fibrin deposition (panel A). Quantification of Immunohistochemistry staining for fibrin on histological sections is also shown and demonstrates reduced fibrin deposition in tPA-FSN treatment group compared to other DIC animals (panel B).
Figure 21 shows clot structure using confocal microscopy (top) and CryoSEM (bottom) of plasma samples from DIC-induced animals treated with saline, FSNs, and tPA-FSNs show improved fiber formation for tPA-FSN treated animals compared to other DIC animals (panel A). Quantification of percent porosity (panel B) and fiber density (panel C) show recovery of clot structure in the tPA-FSN treatment group, similar to wildtype animals.

### DETAILED DESCRIPTION

Before the present methods and systems are disclosed and described, it is to be understood that the methods and systems are not limited to specific synthetic methods, specific components, or to particular compositions. It is also to be understood that the terminology used herein is for the purpose of describing particular embodiments only and is not intended to be limiting.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another embodiment includes, from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Unless started to the contrary, the term "polyacrylamide" includes the unsubstituted polyacrylamide polymer as well as poly(N-alkylacrylamides) and poly(N,N-dialkylacrylamides). The N-alkylacrylamide can be an N-C₁-C₄alkylacrylamide, the N,N-dialkylacrylamide can be an N,N-di(C₁-C₄)alkylacrylamide. The alkyl groups in in the N,N-dialkylacrylamides can be the same, or can be different. The crosslinked polymer can be derived from one or more monomers such as methylacrylamide, ethylacrylamide, n-propylacrylamide, iso-propylacrylamide, n-butylacrylamide, iso-butylacrylamide, sec-butylacrylamide, tert-butylacrylamide, dimethylacrylamide, diethylacrylamide, di-n-propylacrylamide, di-iso-propylacrylamide, N-methyl-N-ethylacrylamide, N-methyl-N-n-propylacrylamide, N-ethyl-N-n-propylacrylamide, N-methyl-N-iso-propylacrylamide, and N-ethyl-N-iso-propylacrylamide. In some instances, the crosslinked polymer is derived from monomers including N-isopropylacrylamide, N-isopropylmethacrylamide, N,N-diethylacrylamide, or a copolymer thereof.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems.

### Platelet-Like Particle (PIP) Nanogels

Disclosed herein are deformable platelet-like particle nanogels that include at least one crosslinked polymer and fibrin-binding moiety. Suitable crosslinked polymers include polyacrylamides, polyacrylates, poly(acrylic acids), polyethylene glycols, polyvinyl alcohols, polysaccharides, polyvinylpyrrolidones, and copolymers thereof. When the crosslinked polymer is a copolymer, it can be a random copolymer or block copolymer.

In certain embodiments, crosslinked polymer can be a copolymer of one or more polyacrylamides (as defined above) and (meth)acrylic acid. As used herein, the term (meth)acrylic acid includes acrylic acid and methacrylic acid. Generally, such copolymers can be prepared from a precipitation polymerization reaction of a mixture of (meth)acrylic acid and suitable acrylamide monomers. The molar ratio of the acrylamide monomer and the (meth)acrylic acid monomer can be from 80:20 to 99:1, from 85:15 to 98:2, from 87.5:12.5 to 95:5, or from 90:10 to 95:5 In some cases, the (meth)acrylic acid component can be present in an amount of no more than 40%, no more than 30%, no more than 20%, no more than 10%, no more than 9%, no more than 8%, no more than 7%, no more than 6%, no more than 5%, no more than 4%, no more than 3%, no more than 2%, or no more than 1% by weight of the total monomer mixture. The precipitation polymerization may be carried out using a free radical initiator such as ammonium persulfate (APS) or 2,2'-azobis(amidinopropane)dihydrochloride.

The precipitation may be carried out using an exogenous crosslinking agent, for instance polyfunctional acrylates and polyfunctional acrylamides such as N,N'-methylenebis(acrylamide), N,N-(1,2-dihydroxyethylene)bisacrylamide, ethylene glycol diacrylate, di(ethylene gycol) diacrylate, tetra(ethylene glycol) diacrylate, ethylene glycol dimethacrylate, di(ethylene glycol) dimethacrylate, and tri(ethylene glycol) dimethacrylate. A preferred crosslinking monomer is *N,N'-*methylenebis(acrylamide).

In other cases, the nanogels disclosed herein can be a shell particle, having a shell of crosslinked monomer enveloping a hollow core. In these cases, the shell can include a crosslinking monomer in a molar amount from 0.1-10%, from 0.5-10%, from 0.5-7.5%, from 1-7.5%, from 1-5%, from 2.5-5%, from 1-2.5%, from 1-2%, from 0.5-2%, or from 1.5-2.5%, relative to the total monomer content in the shell. In some cases, the shell can include a crosslinking monomer in a molar amount no greater than 5%, no greater than 4%, no greater than 3%, no greater than 2%, or no greater than 1%, relative to the total monomer content in the shell.

The shell particles can be obtained from core-shell particles, in which the core is degradable. In some instances, the core has a degradable backbone. The core may also have a non-degradable backbone and crosslinked with a degradable crosslinker. In some instances, both the polymer backbone and crosslinkers can be degradable. Exemplary degradable groups include oxidatively cleavable moieties like vicinal 1,2-diols, olefins and alkynes, reductively cleavable groups like disulfides, and hydrolytically cleavable groups like esters, carbonates, and thioesters. In some embodiments, an enzymatically cleavable peptide sequence can be employed, for instance sequences cleaved by thrombin, plasminogen, MMPs, and others. In certain embodiments, the core can be a polyacrylamide with a degradable crosslinker. Preferable degradable crosslinkers include vicinal diols, which may be oxidatively cleaved using an oxidant such as periodic acid, potassium permanganate, or lead tetraacetate. A preferred vicinal diol is *N,N'*-(1,2-Dihydroxyethylene)bisacrylamide (DHEBA). Any degradable crosslinked polymer may serve as the core, so long as it may be degraded under conditions compatible with the shell.

The hollow PLPs can have a hydrodynamic diameter from 50-1,000 nm, from 50-750 nm, from 100-750 nm, from 150-500 nm, or from 150-350 nm. Hydrodynamic diameter may be determined using Nanosight particle tracking. The hollow PLPs can have a dry particle diameter from 50-1,000 nm, from 50-750 nm, from 100-750 nm, from 150-500 nm, from 150-350 nm, from 25-950 nm, from 25-725 nm, from 75-725 nm, from 125-475 nm, or from 125-325 nm. In some instances, the dry particle diameter is about 90-98%, from 90-95%, from 85-95%, or from 80-90% of the hydrodynamic diameter. Because the disclosed particles are hollow, they exhibit greater deformability relative to non-hollow particles. In some embodiments, the platelet-like particles spread such that their diameter is at least 100x, 250x, 500x, 750x, or 1,000x their height when allowed to dry. Dry particle dimensions (e.g., diameter and height) can be determined using atomic force microscopy.

The nanogels also include at least one fibrin binding moiety, for instance fibrin-binding IgG antibodies, fibrin-binding peptides (fibrin knob mimics), Fragment-D binding antibodies, as well as other antibody fragments than bind fibrin. Exemplary fibrin binding moieties are disclosed in U.S. Publication 2016/0271292 at [0050-0057] and Table 1. In some cases, the fibrin-binding moieties can demonstrate a high affinity for fibrin and negligible binding to soluble fibrinogen.

In some examples, the fibrin biding moieties can be polypeptides encoded by clones A2, A6, and/or H6. In some examples, the fibrin biding moieties can be polypeptides encoded by H6.

The amino acid sequence for H6 is (SEQ ID NO: 1):

QVOLLESGGGLVOPGGSLRLSCAASGDRFTHNDMGWWRQAPGKGLEW
WSTIPTTDGSTYYADSVKGRFTISRDNSKSTLYLOMNSLRAEDTAVYYCA
TGYMWKAPAYWKYWGOGTLWTVSSAAAEOKLISEEDLNSAAHYTDIG.

The amino acid sequence for A2 is (SEQ ID NO: 2):

QVOLLESGGGLVOPGGSLRLSCAASGFSLNDKIMTWVROAPGKGLEWV
SSISGPNGSTYYADSVKGRFTISRDNSKNTLYLOMNSLRAEDTAVYYCAG
CAAASSTMEYWGOGTLVTVSSAAA EOKLISEEDLNSAAHYTDIR.

The amino acid sequence for A6 is (SEQ ID NO: 3):

QVOLLESGGGLVOPGGSLRLSCAASGWRITDDSMSWVROAPGKGLEWV
SSIEDNSGSTYYADSVKGRFTISRDNSKNTLYLOMNSLRAEDTAVYYCAG
EGWDVLRRDOAVTSWGOGTLVTVSSAAAEOKLISEEDLINSAAHYTDMR.

When the crosslinked polymer includes (meth)acrylic acid residues, the fibrin binding moieties may be conjugated using conventional techniques, for instance using standard EDC/NHS chemistry.

The hollow PLPs disclosed herein can include one or more fibrinolytic agents. Suitable agents include fibrinolytic proteins like tissue plasminogen activator (tPA), plasmin, streptokinase, urokinase, as well as derivatized versions of the native protein. Derivatives of the native forms of these proteins can also be used. The fibrinolytic agent can be loaded into the hollow PLP by immersing the hollow PLP in a solution of the fibrinolytic agent. In some embodiments, the concentration of the fibrinolytic agent in the solution is from 1-10,000 µM, from 10-10,000 µm, from 50-5,000 µM, from 100-5,000 µm, from 100-2,500 µM, from 100-1,000 µm, or from 250-1,000 µM. The hollow PLP can be immersed in the solution in an amount from 0.5-1,000 mg/mL, from 1-1,000 mg/mL, from 1-500 mg/mL, from 1-250 mg/mL, from 1-100 mg/mL, from 1-50 mg/mL, from 5-50 mg/mL, from 1-25 mg/mL, from 10-50 mg/mL, from 20-50 mg/mL, or from 15-25 mg/mL.

The hollow PLP can further include one or more small molecule drugs, for instance, a fibrotic inhibitor like Y-27632, a cell contractility inhibitor like HA-1077; EHT1864, NSC23766; latrunculin A, ML-7, PP2, antifungal fibrotic inhibitors like itraconazole, an NSAID like indomethacin, diclofenac, naproxen, and -coxibs such as celecoxib, anti-inflammatory and immunomodulatory drugs, e.g., corticosteroids such as hydrocortisone, prednisone, or dexamethasone. The small molecule drug can be loaded onto the hollow PLP by dissolving it in the same solution as the fibrinolytic agent, for instance at a concentration from 1-10,000 µM, from 10-10,000 µm, from 50-5,000 µM, from 100-5,000 µm, from 100-2,500 µM, from 100-1,000 µm, or from 250-1,000 µM.

When the hollow PLPs are combined with a fibrinolytic agent like tPA, they can be used advantageously in a wide variety of thrombotic events, including myocardial infarction, stroke, limb ischemia, pulmonary embolism, deep vein thrombosis, or hepatic artery thrombosis. The hollow PLP/tPA compositions are also useful to treat disseminated intravascular coagulation. The fibrinolytic hollow PLPs may be administered to a patient by a variety of methods, including intravenously or intramuscularly. The hollow PLPs may be directed applied to a thrombosis or embolism using a catheter.

The nanogels can further include one or more additional therapeutic agents, for instance antimicrobials, analgesics and anti-inflammatories, as well as combinations thereof. In some cases, the therapeutic compound is an antimicrobial agent, e.g., an agent that inhibits the growth of or kill microbes such as bacteria, mycobacteria, viruses, fungi, and parasites. Anti-microbial agents therefore include anti-bacterial agents, anti-mycobacterial agents, anti-viral agents, anti-fungal agents, and anti-parasite agents. Suitable antimicrobials include antibiotics, antimicrobial peptides and metallic compounds. Suitable analgesics include opioids, capsaicin, diclofenac, lidocaine, benzocaine, methyl salicylate, trolamine, prilocaine, pramoxine, dibucaine, phenol, tetracaine, camphor, dyclonine, and menthol. Suitable anti-inflammatories include alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, deflazacort, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lornoxicam, loteprednol etabonate, meclofenamate sodium, meclofenamic acid, meclorisone dibutyrate, mefenamic acid, mesalamine, meseclazone, methylprednisolone suleptanate, morniflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, and zomepirac sodium. In some cases, the therapeutic agent can be a fibrotic inhibitor like Y-27632, a cell contractility inhibitor like HA-1077; EHT1864, NSC23766; latrunculin A, ML-7, PP2, an antifungal fibrotic inhibitor like itraconazole, an NSAID like indomethacin, diclofenac, naproxen, and -coxibs such as celecoxib, anti-inflammatory and immunomodulatory drugs, e.g., corticosteroids such as hydrocortisone, prednisone, or dexamethasone.

In some cases, the therapeutic agent can include at least one growth factor, cytokine, chemokine, cluster differentiation (CD) antigen, neutrophin, hormone, enzyme, viral antigen, bacterial antigen, recombinant protein, natural protein, monoclonal antibody, polyclonal antibody, donor blood serum protein, donor blood plasma protein, or small molecule drug. Exemplary growth factors include keratinocyte growth factor (KGF), platelet derived growth factor (PDGF), transforming growth factor-beta (TGF_{β)}, interleukin, activin, colony stimulating factor, connective tissue growth factor (CTGF), epidermal growth factor (EGF), Epigen, erythropoietin, fibroblast growth factor (FGF), galectin, hepatoma-derived growth factor (HDGF), hepatocyte growth factor, insulin-like growth factor binding protein (IGFBP), insulin-like growth factor, insulin, leptin, macrophage migration inhibitory factor, melanoma inhibitory factor, myostatin, noggin, nephroblastoma overexpressed (NOV), omentin, oncostatinM, osteopontin, osteoprotogerin (OPG), periostin, placenta growth factor, placental lactogen, prolactin, RANK ligand, retinol binding protein, stem cell factor, transforming growth factor, and vascular endothelial growth factor (VEGF). This includes associated isoforms from these growth factor families. In certain preferred embodiments, the nanoparticles described in the above paragraphs can include KGF, interleukin-2 (IL-2), and/or interleukin-6 (IL-6).

Therapeutic agents, such as described above, can be covalently conjugated to the nanoparticle. For instance, the therapeutic agent can be conjugated to the surface of the particle, or can be within the hollow core of the nanoparticle. Therapeutic agents may be conjugated through a linker via the (meth)acrylic residues in the nanogel, or through the fibrin binding moiety. Any suitable linker can be used in accordance with the present invention. Linkers may be used to form amide linkages, ester linkages, disulfide linkages, etc. Linkers may contain carbon atoms or heteroatoms (e.g., nitrogen, oxygen, sulfur, etc.). Typically, linkers are 1 to 50 atoms long, 1 to 40 atoms long, 1 to 25 atoms long, 1 to 20 atoms long, 1 to 15 atoms long, 1 to 10 atoms long, or 1 to 10 atoms long. Linkers may be substituted with various substituents including, but not limited to, hydrogen atoms, alkyl, alkenyl, alkynyl, amino, alkylamino, dialkylamino, trialkylamino, hydroxyl, alkoxy, halogen, aryl, heterocyclic, aromatic heterocyclic, cyano, amide, carbamoyl, carboxylic acid, ester, thioether, alkylthioether, thiol, and ureido groups. As would be appreciated by one of skill in this art, each of these groups may in turn be substituted.

A simple-to-use linker can be an aliphatic or heteroaliphatic linker. For example, the linker can be a polyalkyl linker. The linker can be a polyether linker. The linker can be a polyethylene linker, such as polyethylene glycol (PEG). The linker can be a short peptide chain, e.g., between 1 and 10 amino acids in length, e.g., 1, 2, 3, 4, or 5 amino acids in length, a nucleic acid, an alkyl chain, etc.

The linker can be a cleavable linker. To give but a few examples, cleavable linkers include protease cleavable peptide linkers, nuclease sensitive nucleic acid linkers, lipase sensitive lipid linkers, glycosidase sensitive carbohydrate linkers, pH sensitive linkers, hypoxia sensitive linkers, photo-cleavable linkers, heat-labile linkers, enzyme cleavable linkers (e.g. esterase cleavable linker), ultrasound-sensitive linkers, x-ray cleavable linkers, etc. In some embodiments, the linker is not a cleavable linker.

Any of a variety of methods can be used to associate a linker with a particle and agent. General strategies include passive adsorption (e.g., via electrostatic interactions), multivalent chelation, covalent bond formation, etc. (Gao et al, 2005, Curr. Op. Biotechnol., 16:63). Click chemistry can be used to associate a linker with an agent (e.g. Diels- Alder reaction, Huigsen 1,3 -dipolar cycloaddition, nucleophilic substitution, carbonyl chemistry, epoxidation, dihydroxylation, etc.).

A bifunctional cross-linking reagent can be employed. Such reagents contain two reactive groups, thereby providing a means of covalently associating two target groups. The reactive groups in a chemical cross-linking reagent typically belong to various classes of functional groups such as succinimidyl esters, maleimides, and pyridyldisulfides. Exemplary cross-linking agents include, e.g., carbodiimides, N-hydroxysuccinimidyl-4-azidosalicylic acid (NHS-ASA), dimethyl pimelimidate dihydrochloride (DMP), dimethylsuberimidate (DMS), 3,3'-dithiobispropionimidate (DTBP), N-Succinimidyl 3-[2-pyridyldithio]- propionamido (SPDP), succimidyl α-methylbutanoate , biotinamidohexanoyl-6-amino- hexanoic acid N-hydroxy-succinimide ester (SMCC), succinimidyl-[(N- maleimidopropionamido)-dodecaethyleneglycol] ester (NHS-PEO 12), etc. For example, carbodiimide -mediated amide formation and active ester maleimide-mediated amine and sulfhydryl coupling are widely used approaches.

Common schemes for forming a conjugate involve the coupling of an amine group on one molecule to a thiol group on a second molecule, sometimes by a two- or three- step reaction sequence. A thiol-containing molecule may be reacted with an amine- containing molecule using a heterobifunctional cross-linking reagent, e.g., a reagent containing both a succinimidyl ester and either a maleimide, a pyridyldisulfide, or an iodoacetamide. Amine-carboxylic acid and thiol-carboxylic acid cross-linking, maleimide- sulfhydryl coupling chemistries (e.g., the maleimidobenzoyl-N-hydroxysuccinimide ester (MBS) method), etc., may be used. Polypeptides can conveniently be attached to particles via amine or thiol groups in lysine or cysteine side chains respectively, or by an N-terminal amino group. Nucleic acids such as RNAs can be synthesized with a terminal amino group. A variety of coupling reagents (e.g., succinimidyl 3-(2-pyridyldithio)propionate (SPDP) and sulfosuccinimidyl-4-(N-maleimidomethyl)cyclohexane-1-carboxylate (sulfo-SMCC) may be used to associate the various components of conjugates. Agents can be prepared with functional groups, e.g., amine or carboxyl groups, available at the surface to facilitate association with a biomolecule. Any biomolecule can be attached to another molecule described herein using any of the methods described herein.

In further embodiments, a therapeutic agent can be entrapped or encapsulated within the nanogel, for instance, not covalently bonded to (meth)acrylic acid residues or fibrin binding molecules. The therapeutic agent can be loaded into the nanogel by immersing the dried nanogels in a solution of the agent, for instance at a concentration from 1-10,000 µM, from 10-10,000 µm, from 50-5,000 µM, from 100-5,000 µm, from 100-2,500 µM, from 100-1,000 µm, or from 250-1,000 µM.

Also disclosed are methods of treating a wound by administering the PLPs described above to the site the wound. Exemplary wounds that may be treated with the PLPs include a trauma wound, a surgical wound, a burn wound, or an ulcer wound. Wound patients with coagulation disorders may be advantageously treated with the PLPs, for instance, diabetics, heniophiliaes, patients with vitamin K deficiency, Von Willebrand disease or other clotting factor deficiencies. The PLPs can be used to treat wounds in patients undergoing anti-coagulation therapy, for instance patients receiving heparin, fandaparinux, idraparinux, vitamin K, cotimadin, direct thrombin inhibitors like argatroban, dabigatran, factor Xa inhibitors like rivaroxaban, apixaban and edoxaban, anti-platelet agents such as clopidogrel and prasugrel. The PLPs can be used to treat wounds arising from medical procedures such as percutaneous coronary intervention, stem and/or valve placement or repair, transfusion, and dialysis.

The nanogels described above can be included in a wide variety of pharmaceutical compositions, for instance those including pharmaceutically acceptable carriers. Suitable carriers include water, saline and other liquid formulations, which can be directly administered to a wound site or injected into a patient. In other cases, the nanogels can be included in a formulation for topical administration, for instance, lotions, sprays, creams, ointments and the like. The compositions can also include a backing layer to secure the composition at a wound site.

The pharmaceutical compositions described herein may be prepared by any method known or hereafter developed in the art of pharmaceutics. In general, such preparatory methods include the step of bringing the active ingredient into association with one or more excipients and/or one or more other accessory ingredients, and then, if necessary and/or desirable, shaping and/or packaging the product into a desired single- or multi-dose unit.

Dosage forms for topical and/or transdermal administration of the nanoparticles may include ointments, pastes, creams, lotions, gels, powders, solutions, sprays, inhalants and/or patches. Generally, the active component is admixed under sterile conditions with a pharmaceutically acceptable excipient and/or any needed preservatives and/or buffers as may be required. Additionally, the present invention contemplates the use of transdermal patches, which often have the added advantage of providing controlled delivery of an active ingredient to the body. Such dosage forms may be prepared, for example, by dissolving and/or dispensing the active ingredient in the proper medium. Alternatively or additionally, the rate may be controlled by either providing a rate controlling membrane and/or by dispersing the active ingredient in a polymer matrix and/or gel.

Suitable devices for use in delivering intradermal pharmaceutical compositions described herein include short needle devices such as those described in U.S. Patents 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof. Jet injection devices which deliver liquid compositions to the dermis via a liquid jet injector and/or via a needle which pierces the stratum corneum and produces a jet which reaches the dermis are suitable. Jet injection devices are described, for example, in U.S. Patents 5,480,381; 5,599,302; 5,334,144; 5,993,412; 5,649,912; 5,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the excipients and/or additional ingredients described herein.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in the inventive formulations. Excipients such as cocoa butter and suppository waxes, coloring agents, coating agents, sweetening, flavoring, and perfuming agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, etc., and combinations thereof

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation- exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, etc., and combinations thereof.

Exemplary surface active agents and/or emulsifiers include, but are not limited to, natural emulsifiers (e.g. acacia, agar, alginic acid, sodium alginate, tragacanth, chondrux, cholesterol, xanthan, pectin, gelatin, egg yolk, casein, wool fat, cholesterol, wax, and lecithin), colloidal clays (e.g. bentonite [aluminum silicate] and Veegum [magnesium aluminum silicate]), long chain amino acid derivatives, high molecular weight alcohols (e.g. stearyl alcohol, cetyl alcohol, oleyl alcohol, triacetin monostearate, ethylene glycol distearate, glyceryl monostearate, and propylene glycol monostearate, polyvinyl alcohol), carbomers (e.g. carboxy polymethylene, polyacrylic acid, acrylic acid polymer, and carboxyvinyl polymer), carrageenan, cellulosic derivatives (e.g. carboxymethylcellulose sodium, powdered cellulose, hydroxymethyl cellulose, hydroxypropyl cellulose, hydroxypropyl methylcellulose, methylcellulose), sorbitan fatty acid esters (e.g. polyoxyethylene sorbitan monolaurate [Tween 20], polyoxy ethylene sorbitan [Tween 60], polyoxy ethylene sorbitan monooleate [Tween 80], sorbitan monopalmitate [Span 40], sorbitan monostearate [Span 60], sorbitan tristearate [Span 65], glyceryl monooleate, sorbitan monooleate [Span 80]), polyoxyethylene esters (e.g. polyoxyethylene monostearate [Myrj 45], polyoxyethylene hydrogenated castor oil, polyethoxylated castor oil, polyoxymethylene stearate, and Solutol), sucrose fatty acid esters, polyethylene glycol fatty acid esters (e.g. Cremophor), polyoxyethylene ethers, (e.g. polyoxyethylene lauryl ether [Brij 30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic F 68, Poloxamer 188, cetrimonium bromide, cetylpyridinium chloride, benzalkonium chloride, docusate sodium, etc. and/or combinations thereof.

Exemplary binding agents include, but are not limited to, starch (e.g. cornstarch and starch paste); gelatin; sugars (e.g. sucrose, glucose, dextrose, dextrin, molasses, lactose, lactitol, mannitol); natural and synthetic gums (e.g. acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminum silicate (Veegum), and larch arabogalactan); alginates; polyethylene oxide; polyethylene glycol; inorganic calcium salts; silicic acid; polymethacrylates; waxes; water; alcohol; etc.; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl. In certain embodiments, the preservative is an anti-oxidant. In other embodiments, the preservative is a chelating agent.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, etc., and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, etc., and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

Liquid dosage forms for oral and parenteral administration include, but are not limited to, pharmaceutically acceptable emulsions, microemulsions, solutions, suspensions, syrups and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and perfuming agents. In certain embodiments for parenteral administration, conjugates can be mixed with solubilizing agents such as Cremophor, alcohols, oils, modified oils, glycols, polysorbates, cyclodextrins, polymers, and combinations thereof.

Injectable preparations, for example, sterile injectable aqueous or oleaginous suspensions, may be formulated according to the known art using suitable dispersing or wetting agents and suspending agents. The sterile injectable preparation may be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U. S. P. and isotonic sodium chloride solution, etc. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

In order to prolong the effect of an active ingredient, it is often desirable to slow the absorption of the active ingredient from subcutaneous or intramuscular injection. This may be accomplished by the use of a liquid suspension of crystalline or amorphous material with poor water solubility. The rate of absorption of the active ingredient then depends upon its rate of dissolution which, in turn, may depend upon crystal size and crystalline form. In some embodiments, delayed absorption of a parenterally administered active ingredient is accomplished by dissolving or suspending the drug in an oil vehicle.

Compositions for rectal or vaginal administration are typically suppositories which can be prepared by mixing the conjugates with suitable non-irritating excipients such as cocoa butter, polyethylene glycol or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable excipient such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets and pills, the dosage form may comprise buffering agents.

Solid compositions of a similar type may be employed as fillers in soft and hard- filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type may be employed as fillers in soft and hard- filled gelatin capsules using such excipients as lactose or milk sugar as well as high molecular weight polyethylene glycols and the like.

Spray or aerosol formulations may include one or more propellants. Low boiling propellants generally include liquid propellants having a boiling point of below 65°F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic and/or solid anionic surfactant and/or a solid diluent (which may have a particle size of the same order as particles comprising the active ingredient).

Pharmaceutical compositions formulated for pulmonary delivery may provide the active ingredient in the form of droplets of a solution and/or suspension. Such formulations may be prepared, packaged, and/or sold as aqueous and/or dilute alcoholic solutions and/or suspensions, optionally sterile, comprising the active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. The droplets provided by this route of administration may have an average diameter in the range from about 0.1 µm to about 200 µm.

### Core-Shell Nanogels

Also provided are fibrin-specific core-shell nanogels. The fibrin-specific nanogels can be core-shell particles comprising a core comprising a first crosslinked polymer network; and a shell comprising a second crosslinked polymer network at least partially surrounding the core. In some embodiments, the shell can completely envelope the core. The first crosslinked polymer network, the second crosslinked polymer network, or a combination thereof can comprise a crosslinked polyacrylamide matrix. In some embodiments, the crosslinking density of the core can be different than the crosslinking density of the shell (e.g., the crosslinking density of the core can be greater than the crosslinking density of the shell). A fibrin binding moiety is conjugated to the polymeric matrix (e.g., the crosslinked polyacrylamide matrix) forming the shell.

Suitable crosslinked polymers include polyacrylamides, polyacrylates, poly(acrylic acids), polyethylene glycols, polyvinyl alcohols, polysaccharides, polyvinylpyrrolidones, and copolymers thereof. When the crosslinked polymer is a copolymer, it can be a random copolymer or block copolymer.

In certain embodiments, crosslinked polymer can be a copolymer of one or more polyacrylamides (as defined above) and (meth)acrylic acid. As used herein, the term (meth)acrylic acid includes acrylic acid and methacrylic acid. Generally, such copolymers can be prepared from a precipitation polymerization reaction of a mixture of (meth)acrylic acid and suitable acrylamide monomers. The molar ratio of the acrylamide monomer and the (meth)acrylic acid monomer can be from 80:20 to 99:1, from 85:15 to 98:2, from 87.5:12.5 to 95:5, or from 90:10 to 95:5 In some cases, the (meth)acrylic acid component can be present in an amount of no more than 40%, no more than 30%, no more than 20%, no more than 10%, no more than 9%, no more than 8%, no more than 7%, no more than 6%, no more than 5%, no more than 4%, no more than 3%, no more than 2%, or no more than 1% by weight of the total monomer mixture. The precipitation polymerization may be carried out using a free radical initiator such as ammonium persulfate (APS) or 2,2'-azobis(amidinopropane)dihydrochloride.

The precipitation may be carried out using an exogenous crosslinking agent, for instance polyfunctional acrylates and polyfunctional acrylamides such as *N*,*N*'-methylenebis(acrylamide), N,N-(1,2-dihydroxyethylene)bisacrylamide, ethylene glycol diacrylate, di(ethylene gycol) diacrylate, tetra(ethylene glycol) diacrylate, ethylene glycol dimethacrylate, di(ethylene glycol) dimethacrylate, and tri(ethylene glycol) dimethacrylate. A preferred crosslinking monomer is *N,N'-*methylenebis(acrylamide).

In some cases, the nanogels disclosed herein can be a core shell particle, having a core of one crosslinked polymer or copolymer, and a shell of a different polymer or copolymer. In some cases, both the core and the shell can have the same crosslinked polymer, but the crosslinking density in the core will be different from the crosslinking density of the shell. In some embodiments, the core will be more densely crosslinked than the shell. For instance, the core can include a crosslinking monomer in a molar amount from 1-20%, from 2.5-20%, from 5-20%, from 1-15%, from 2-15%, from 5-15%, from 7.5-15%, or from 7.5-12.5%, relative to the total monomer content in the core. The shell can include a crosslinking monomer in a molar amount from 0.1-10%, from 0.5-10%, from 0.5-7.5%, from 1-7.5%, from 1-5%, or from 2.5-5%, relative to the total monomer content in the shell.

The core-shell nanogels also include at least one fibrin binding moiety, for instance fibrin-binding IgG antibodies, fibrin-binding peptides (fibrin knob mimics), Fragment-D binding antibodies, as well as other antibody fragments than bind fibrin. Exemplary fibrin binding moieties are disclosed in U.S. Publication 2016/0271292 at [0050-0057], and are discussed above. When the crosslinked polymer includes (meth)acrylic acid residues, the fibrin binding moieties may be conjugated using conventional techniques, for instance using standard EDC/NHS chemistry.

Optionally, the core-shell nanogels disclosed herein can include one or more fibrinolytic agents. Suitable agents include fibrinolytic proteins like tissue plasminogen activator (tPA), plasmin, streptokinase, urokinase, as well as derivatized versions of the native protein. Derivatives of the native forms of these proteins can also be used. The fibrinolytic agent can be loaded into the nanogel by immersing the nanogel in a solution of the fibrinolytic agent. In some embodiments, the concentration of the fibrinolytic agent in the solution is from 1-10,000 µM, from 10-10,000 µm, from 50-5,000 µM, from 100-5,000 µm, from 100-2,500 µM, from 100-1,000 µm, or from 250-1,000 µM. The nanogel can be immersed in the solution in an amount from 0.5-1,000 mg/mL, from 1-1,000 mg/mL, from 1-500 mg/mL, from 1-250 mg/mL, from 1-100 mg/mL, from 1-50 mg/mL, from 5-50 mg/mL, from 1-25 mg/mL, from 10-50 mg/mL, from 20-50 mg/mL, or from 15-25 mg/mL.

Optionally, the core-shell nanogels can further include one or more small molecule drugs, for instance, a fibrotic inhibitor like Y-27632, a cell contractility inhibitor like HA-1077; EHT1864, NSC23766; latrunculin A, ML-7, PP2, an antifungal fibrotic inhibitors like itraconazole, an NSAID like indomethacin, diclofenac, naproxen, and -coxibs such as celecoxib, an anti-inflammatory and immunomodulatory drugs, e.g., a corticosteroids such as hydrocortisone, prednisone, or dexamethasone. The small molecule drug can be loaded onto the nanogel by dissolving it in the same solution as the fibrinolytic agent (when present), for instance at a concentration from 1-10,000 µM, from 10-10,000 µm, from 50-5,000 µM, from 100-5,000 µm, from 100-2,500 µM, from 100-1,000 µm, or from 250-1,000 µM.

### Fibrinolytic Nanogels

Also provided are fibrinolytic nanogels. The fibrinolytic gels described herein can include at least one crosslinked polymer, a fibrin-binding moiety, and a fibrinolytic agent, for instance a fibrinolytic protein such as a tissue plasminogen activator (tPA). In the invention, the fibrinolytic nanogel is hollow particles, and the fibrinolytic protein is tPA. Suitable crosslinked polymers include polyacrylamides, polyacrylates, poly(acrylic acids), polyethylene glycols, polyvinyl alcohols, polysaccharides, polyvinylpyrrolidones, and copolymers thereof. When the crosslinked polymer is a copolymer, it can be a random copolymer or block copolymer.

In certain embodiments, crosslinked polymer can be a copolymer of one or more polyacrylamides (as defined above) and (meth)acrylic acid. As used herein, the term (meth)acrylic acid includes acrylic acid and methacrylic acid. Generally, such copolymers can be prepared from a precipitation polymerization reaction of a mixture of (meth)acrylic acid and suitable acrylamide monomers. The molar ratio of the acrylamide monomer and the (meth)acrylic acid monomer can be from 80:20 to 99:1, from 85:15 to 98:2, from 87.5:12.5 to 95:5, or from 90:10 to 95:5 In some cases, the (meth)acrylic acid component can be present in an amount of no more than 40%, no more than 30%, no more than 20%, no more than 10%, no more than 9%, no more than 8%, no more than 7%, no more than 6%, no more than 5%, no more than 4%, no more than 3%, no more than 2%, or no more than 1% by weight of the total monomer mixture. The precipitation polymerization may be carried out using a free radical initiator such as ammonium persulfate (APS) or 2,2'-azobis(amidinopropane)dihydrochloride.

The precipitation may be carried out using an exogenous crosslinking agent, for instance polyfunctional acrylates and polyfunctional acrylamides such as *N*,*N*'-methylenebis(acrylamide), N,N-(1,2-dihydroxyethylene)bisacrylamide, ethylene glycol diacrylate, di(ethylene gycol) diacrylate, tetra(ethylene glycol) diacrylate, ethylene glycol dimethacrylate, di(ethylene glycol) dimethacrylate, and tri(ethylene glycol) dimethacrylate. A preferred crosslinking monomer is *N,N'-*methylenebis(acrylamide).

In some cases, the nanogels disclosed herein can be a core shell particle, having a core of one crosslinked polymer or copolymer, and a shell of a different polymer or copolymer. In some cases, both the core and the shell can have the same crosslinked polymer, but the crosslinking density in the core will be different from the crosslinking density of the shell. In some embodiments, the core will be more densely crosslinked than the shell. For instance, the core can include a crosslinking monomer in a molar amount from 1-20%, from 2.5-20%, from 5-20%, from 1-15%, from 2-15%, from 5-15%, from 7.5-15%, or from 7.5-12.5%, relative to the total monomer content in the core. The shell can include a crosslinking monomer in a molar amount from 0.1-10%, from 0.5-10%, from 0.5-7.5%, from 1-7.5%, from 1-5%, or from 2.5-5%, relative to the total monomer content in the shell.

The fibrinolytic nanogels also include at least one fibrin binding moiety, for instance fibrin-binding IgG antibodies, fibrin-binding peptides (fibrin knob mimics), Fragment-D binding antibodies, as well as other antibody fragments than bind fibrin. Exemplary fibrin binding moieties are disclosed in U.S. Publication 2016/0271292 at ¶¶ [0050-0057], and are discussed above. When the crosslinked polymer includes (meth)acrylic acid residues, the fibrin binding moieties may be conjugated using conventional techniques, for instance using standard EDC/NHS chemistry.

The nanogels disclosed herein can include one or more fibrinolytic agents. Suitable agents include fibrinolytic proteins like tissue plasminogen activator (tPA), plasmin, streptokinase, urokinase, as well as derivatized versions of the native protein. Derivatives of the native forms of these proteins can also be used. The fibrinolytic agent can be loaded into the nanogel by immersing the nanogel in a solution of the fibrinolytic agent. In some embodiments, the concentration of the fibrinolytic agent in the solution is from 1-10,000 µM, from 10-10,000 µm, from 50-5,000 µM, from 100-5,000 µm, from 100-2,500 µM, from 100-1,000 µm, or from 250-1,000 µM. The nanogel can be immersed in the solution in an amount from 0.5-1,000 mg/mL, from 1-1,000 mg/mL, from 1-500 mg/mL, from 1-250 mg/mL, from 1-100 mg/mL, from 1-50 mg/mL, from 5-50 mg/mL, from 1-25 mg/mL, from 10-50 mg/mL, from 20-50 mg/mL, or from 15-25 mg/mL.

The nanogel can further include one or more small molecule drugs, for instance, a fibrotic inhibitor like Y-27632, a cell contractility inhibitor like HA-1077; EHT1864, NSC23766; latrunculin A, ML-7, PP2, an antifungal fibrotic inhibitors like itraconazole, an NSAID like indomethacin, diclofenac, naproxen, and -coxibs such as celecoxib, an anti-inflammatory and immunomodulatory drugs, e.g., a corticosteroids such as hydrocortisone, prednisone, or dexamethasone. The small molecule drug can be loaded onto the nanogel by dissolving it in the same solution as the fibrinolytic agent, for instance at a concentration from 1-10,000 µM, from 10-10,000 µm, from 50-5,000 µM, from 100-5,000 µm, from 100-2,500 µM, from 100-1,000 µm, or from 250-1,000 µM.

The fibrinolytic nanogels can be used advantageously in a wide variety of thrombotic events, including myocardial infarction, stroke, limb eschemia, pulmonary embolism, deep vein thrombosis, or hepatic artery thrombosis. The nanogels are also useful to treat disseminated intravascular coagulation. The fibrinolytic nanogels may be administered to a patient by a variety of methods, including intravenously or intramuscularly. The nanogels may be directed applied to a thrombosis or embolism using a catheter. In further embodiments, the nanogel may be lyophilized and admixed with one or more pharmaceutical carriers, and formulated into ointments, lotions, creams, gels, drops, suppositories, sprays, liquids and powders.

By way of non-limiting illustration, examples of certain embodiments of the present disclosure are given below.

### EXAMPLES

### Example 1: Biomimetic Microgels with Controllable Deformability Improve Healing outcomes.

Platelets mediate hemostasis by aggregating and binding to fibrin to promote clotting. Over time, platelets contract the fibrin network to induce clot retraction, which contributes to wound healing outcomes by increasing clot stability and improving blood flow to ischemic tissue. In this example, hollow platelet-like particles (PLPs) are described that mimic the native platelet function of clot retraction in a controlled manner and demonstrate that clot retraction-inducing PLPs promote healing *in vivo.* PLPs can be created by coupling fibrin-binding antibodies to CoreShell (CS) or hollow N-isopropylacrylamide (NIPAm) microgels with varying degrees of shell crosslinking. Hollow microgels with loosely crosslinked shells can display a high degree of deformability and mimic activated platelet morphology, while intact CS microgels and hollow microgels with increased crosslinking in the shell do not. When coupled to a fibrin-binding antibody to create PLPs, hollow particles with low degrees of shell crosslinking cause fibrin clot collapse *in vitro,* recapitulating the clot retraction function of platelets, while other particle types do not. Furthermore, hollow PLPs with low degrees of shell crosslinking improve some wound healing outcomes *in vivo.*

### Background

Wound repair initiates at the onset of injury with the formation of a provisional fibrin mesh to cease bleeding and promote tissue remodeling by stimulating cell migration, angiogenesis, and matrix production. Platelets play a critical role in the wound healing process through their roles in hemostasis, clot retraction, and immune modulation. In the early stages of wound repair, platelets aggregate and promote fibrin polymerization to form the primary clot and stem hemorrhage. After cessation of bleeding, platelets induce fibrin clot retraction over several hours. Clot retraction significantly decreases clot size, alters clot organization and increases clot stiffness, thereby promoting ongoing wound repair. Both intrinsic and medication-induced platelet dysfunction beyond hemostasis are associated with non-healing wounds; therefore, synthetic platelet therapies that reproduce the hemostatic and clot retraction features of native platelets could be effective therapies for treating non-healing wounds.

Due to the fundamental role of platelets in hemostasis, there has been great interest in developing synthetic platelets that interact with the native coagulation cascade to promote clotting and treat traumatic injury. A number of platelet-mimetic materials have been developed to reproduce the hemostatic properties of platelets. Overall, approaches to synthetic platelet design focus on replicating various platelet functions. One such example mimics platelet morphology to recreate platelet margination. Other synthetic platelet designs incorporate a nanoparticle decorated with binding domains to mimic platelet binding to coagulation components. Some notable examples include RGD-PLL-PLGA particles, albumin microparticles coated with fibrinogen, and liposomal particles conjugated with GPIb. Recent studies have also demonstrated that synthetic platelets encompassing collagen-binding and vWF-binding motifs improve binding to wound sites under flow conditions. Injectable, shear-thinning composite hydrogels containing gelatin and silicate nanoplatelets have also been described for use as an embolic agent for endovascular embolization procedures.

A different approach to developing platelet mimetic materials to augment hemostasis has been demonstrated using highly deformable poly(N-isopropylacrylamide) (pNIPAm) hydrogels (microgels). When coupled to high affinity fibrin-binding antibodies to target the wound environment, these PLPs can mimic natural platelet functions to bind the wound site, stabilize clot structure, and enhance clot formation. PLPs have been previously evaluated for hemostatic effectiveness *in vitro* using a microfluidics device that emulates microvasculature; these studies showed PLPs induce clot formation. Additionally, PLPs honed to injury sites and augmented clotting *in vivo* in a rodent injury model. Perhaps most excitingly, this unique PLP design has been shown to mimic native platelet clot retraction. The high degree of particle deformability of the microgel body, along with high fibrin affinity of the conjugated fibrin antibody, facilitates a Brownian wrench mechanism that induces clot retraction.

PLP-induced clot retraction is an important feature for promoting clot stability. It is postulated that like native platelets, PLP-induced clot retraction could be used to enhance wound repair. The PLP design utilized a highly deformable, ultra-low crosslinked (ULC) microgel that, when coupled to a fibrin-binding single domain variable fragment (sdFv) antibody, induced clot retraction; highly crosslinked fibrin-binding microgels did not induce clot retraction due to decreased particle deformability. In this example, a hollow microgel structure is described that provides a high level of control over particle deformability and thereby allows for previously unachieved control over clot retraction events that are critical to hemostasis and wound healing. The ability to control the timing of clot retraction by "switching on" particle deformability is also described. This can be achieved by synthesizing CoreShell (CS) microgels with degradable cores. Upon core degradation, hollow microgels would display high degrees of deformability, mimic platelet morphology, and induce fibrin-clot collapse when coupled to fibrin-binding motifs in a manner similar to ULC-based PLPs. It was also hypothesized that PLP-mediated clot retraction, like native platelet-mediated clot retraction, would enhance healing responses following injury. To explore this hypothesis, the effect of microgel architecture (CS vs. hollow) and shell crosslinking density on particle deformability and the ability to mimic native activated platelet morphology was characterized. The CS or hollow microgels were then conjugated to fibrin-binding antibodies to create PLPs and analyzed the effect of particle architecture and shell crosslinking on clot retraction *in vitro.* Finally, an *in vivo* murine full-thickness dermal wound model was employed to compare topical application of CS PLPs, hollow PLPs, or saline on wound healing. Collectively, the results demonstrate that 1) platelet-mimetic materials can be created that facilitate temporal control over clot retraction and 2) platelet-mimetic materials that induce clot retraction can enhance some healing outcomes beyond hemostasis.

### Hollow microgels with low degrees of crosslinking in the shell mimic activated platelet morphology.

To explore the hypothesis that hollow microgels will display high degrees of deformability and mimic activated platelet morphology, CS microgels were synthesized by encasing a degradable microgel core with an outer microgel shell with varying degrees of crosslinking. Size characterization of CS microgels was quantified using Dynamic Light Scattering (DLS) (Table 1), where an increase in BIS crosslinker in the shell resulted in a smaller hydrodynamic diameter, likely due to higher levels of core compaction. Hollow particles formed after core dissolution showed an approximate 2-fold increase in hydrodynamic diameter compared to CS microgels. The morphology of intact CS microgels or hollow microgels was then compared to that of resting or active platelets using cryogenic scanning electron microscopy (CryoSEM) with a JEOL 7600F at 50000X magnification (Figure 1). Native circulating platelets display an ovoid morphology and form spindle-like projections upon activation. This was confirmed with isolated resting human platelets and platelets activated with 0.25 U/mL human α-thrombin. CryoSEM revealed that CS microgels displayed a morphology similar to native inactive platelets, while hollow microgels with loosely crosslinked shells displayed morphologies similar to activated platelets with spindle-like projections (Figure 1). Further characterization of deformability was performed under dry conditions using AFM imaging of microgels spread on a glass surface. Microgel diameter increased and height decreased as BIS crosslinker percentage decreased, indicating higher degrees of deformability in less crosslinked microgels. Studies with single-layer lightly self-crosslinked pNIPAm ULCs were observed to be highly deformable and spread extensively on surfaces, while the addition of crosslinking in the form of 2-7% BIS resulted in loss of particle deformability. The results here suggest that due to their architecture, hollow microgels display high levels of deformability similar to ULC single layer microgels. However, this deformability diminishes with increasing crosslinking in the shell. The 2% BIS hollow microgels most closely mimicked activated platelet morphology with spindle-like projections, and demonstrated greater deformability spreading 2.8 times greater than 2% BIS CS microgels, and were therefore used in subsequent *in vitro* and *in vivo* experiments.

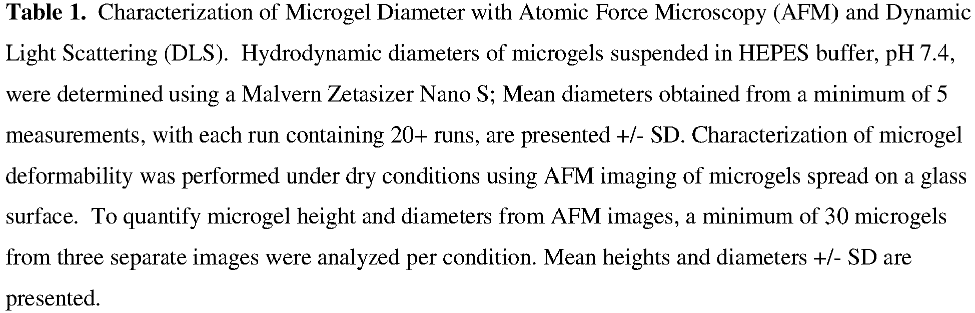

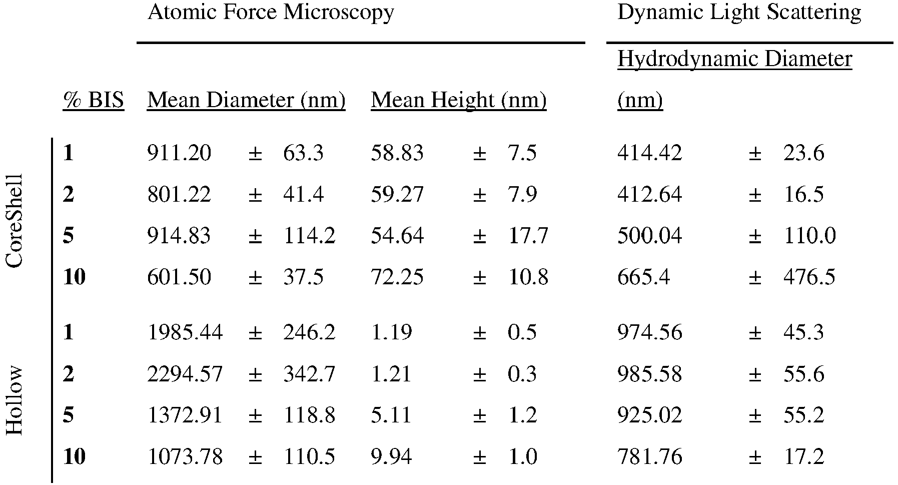

### Hollow microgels with low degrees of crosslinking in the shell recapitulate platelet-mediated clot retraction.

The influence of particle architecture on the ability of PLPs to induce clot retraction was investigated. Because of their high degree of deformability and morphological similarity to activated platelets, it was hypothesized that PLPs constructed from hollow 2% microgels would induce clot retraction while intact CS microgel PLPs would not. This rationale is based on studies which demonstrated PLPs constructed from highly deformable ULC microgels are able to spread extensively within fibrin networks and, due to a Brownian wrench mechanism, induce deformations within the fibrin network. This mechanism was characterized using a combination of *in silico* and *in vitro* experiments and it was found that the high degree of particle deformability and high fibrin binding affinity allows PLPs to spread and interact extensively within fibrin networks. Over time, the particles return to a spherical shape but maintain their adhesion to the fibrin fibers, thereby inducing microcollapses within the network. *In silico* experiments demonstrated that increased particle deformability and strength of adhesion to the fibrin network decreased the time scale and increased the degree of collapse. Indeed, it was observed that hollow fibrin-binding microgels with 2% BIS crosslinking in the shell can induce a fibrin clot collapse similar to clot collapse observed with native platelets. Figure 2 demonstrates fibrin clot collapse is dependent on particle deformability, where less deformable CS microgel PLPs did not significantly affect fibrin ultrastructure. The ability of hollow PLPs to induce clot retraction is likely due to the morphology of the hollow microgels, which allows the particles to spread within and interface extensively within the fibrillar fibrin network. Ultimately, these particles return to a spherical shape while maintaining connections with the fibrin network, thereby inducing microcollapses. This theory is supported by *in silico* studies with deformable ULC-based PLPs. CS PLPs do not exhibit these behaviors because they are more rigid due to the high degree of crosslinking in the core. This phenomenon's dependence on deformability was again emphasized *in situ* where core degradation induced gross fibrin clot collapse (Figure 2, panel B). Here, *in situ* degradation of the microgel core was investigated to determine if degradation would result in inducible clot retraction. Fibrin clots were formed in the presence of intact CS PLPs. Following polymerization, sodium periodate (NaIO₄) was added to the clots to induce core degradation. After 48 hours, a decrease in clot size was observed compared to the fibrin only control. However, the decrease in clot size was not as robust as in the presence of pre-degraded, hollow PLPs. Additionally, it should be noted that because this mechanism is driven by Brownian motion, resulting time scales are slower than those for clot retraction by native platelets.

### Hollow PLPs enhance wound healing outcomes in vivo.

Because native platelet-mediated clot retraction has been shown to enhance wound healing outcomes by decreasing clot size, altering clot organization, and improving reperfusion to the tissue, it was hypothesized that hollow PLPs capable of inducing clot retraction would likewise enhance wound healing responses. Wound healing was investigated in a mouse full thickness dermal injury model treated with hollow PLPs, CS PLPs or saline. Hollow PLPs enhanced some wound healing outcomes compared to CS PLPs or saline controls (Figure 3). Histology of wound cross-sections at day 8 with Martius Scarlet Blue (MSB) staining revealed an increase in fibrin deposition and an intact, significantly thicker epidermis (p<0.01) in hollow PLP treatment compared to saline controls or CS PLPs treatment. A trend of enhanced healing in the presence of hollow PLPs was observed. IHC of wound cross-sections showed a significant increase in CD31+ vessel numbers (p<0.001 hollow compared to saline or CS PLPs; Figure 3), an endothelial marker indicating angiogenesis, which is a critical step in wound healing, as well as an increase in proliferation marker Ki67. These combined results demonstrate topical application of deformable hollow PLPs improve some wound healing outcomes in a murine full thickness model to a greater extent than less deformable CS PLPs or saline. While significant differences were observed in histological measures, it should be noted that statistical differences were not observed in percent wound closure values. This is likely due to the small sample size and the small initial wound areas used and is a limitation of the study.

In an *in vivo* model, functional endogenous platelets are expected to contribute to wound healing; therefore, the comparison between hollow PLPs and saline demonstrates enhanced healing over that observed in the presence of native platelets (i.e., the saline control). The application of platelet rich plasma (PRP), which delivers additional native platelets to the wound bed, significantly decreased wound sizes in mouse full thickness injury models, similar to the one utilized in this example. Specifically, PRP decreased wound size after 10 days by ~20% compared to saline treated animals, which is similar to our observations (Figure 3).

### Conclusions

In conclusion, hollow microgels consisting of loosely crosslinked pNIPAm-BIS-AAc shells created by dissolving a degradable core show significant potential to assume key functions of endogenous platelets. These synthetic particles are able to mimic platelet morphology and, when coupled to fibrin-binding motifs, can function to mediate fibrin clot retraction in a manner similar to native platelets. Native platelets within a clot bind fibrin fibers, spread within and actively modify the fibrin network, leading to clot retraction; this results in increased fibrin density and matrix stiffness. This increased fibrin density and clot stiffness contributes to enhanced healing through a number of mechanisms including enhanced clot stability and increased resistance to fibrinolysis. Native platelets are capable of generating great contractile forces upon activation and, combined with their high elasticity, are capable of stiffening clots in conjunction with strain stiffening of fibrin under tension due to platelet contraction. Platelet cytoskeletal motility proteins and Factor 13 (FXIIIa) mediate clot structure and bring about a differential structural gradient, with platelets concentrated at the clot perimeter and close-packed polyhedral erythrocytes retained in the center. The ability to mimic and controllably induce clot retraction in a synthetic platelet platform can be used to mimic these platelet functions and promote healing beyond the initial cessation of bleeding.

The hollow PLP architecture and composition described herein can be conjugated to a fibrin-binding motif and tuned to achieve the appropriate degree of deformability necessary to reproduce the mechanical function of clot retraction. 2% BIS hollow microgels showed platelet-like morphology and clot contraction and were tested *in vivo* in a rodent model of dermal wound repair, revealing improved histological outcomes compared to saline controls. Microgel-mediated clot retraction has been shown to be dependent upon a microgel's 1) fibrin-binding ability and 2) deformability (ability to spread on a glass surface). 2% BIS CoreShell PLPs can bind fibrin, however they lack the deformability necessary to induce a clot retraction, as seen in confocal microscopy images in Figure 2, panel A. When the densely crosslinked NIPMAm-DHEBA core is degraded with NaIO₄, however, a deformable hollow pNIPAm-BIS shell remains, which facilitates fibrin network deformation (Figure 2, panel A). Deformability can be "switched on" through core degradation following incorporation of CoreShell PLPs into fibrin clots, creating hollow PLPs, which are then capable of inducing clot retraction as demonstrated in the images presented in Figure 2, panel B. The ability to "switch on" deformability which triggers clot retraction can allow for temporal control over induction of clot retraction. In conclusion, hollow fibrin-binding microgels are biomimetic and are a promising platform for promoting wound repair.

### Experimental Section

**CoreShell & Hollow Microgel Synthesis.** CS microgels were constructed by first producing a core containing *N*-isopropylmethacrylamide (NIPMAm) and crosslinked with *N,N'*-(1,2-Dihydroxyethylene)bisacrylamide (DHEBA) (Sigma) through a precipitation polymerization reaction. NIPMAm was recrystallized with hexanes. NIPMAm and DHEBA (90% and 10% of a 140 mM monomer solution by weight respectively) in ultrapure water were filtered over a 0.22 µm membrane into a 3-neck reaction vessel heated in silicon oil, stirred at 450 rpm, and fluxed at 70°C for 1 hr under nitrogen. 1 mM ammonium persulfate (APS) was added to initiate the reaction. After 6 hours, cores were purified by filtering over glass wool to remove large aggregates and by dialysis (1000 kDa MWCO tubing (Spectrum)) against ultrapure water for 2 days. Cores were enveloped within shells in a second precipitation polymerization reaction. A solution containing N-Isopropylacrylamide (NIPAm) and N,N'-Methylenebisacrylamide (BIS) was dissolved in ultrapure water and filtered. The solution was mixed with purified cores in a volume ratio of 60% shell to 40% core solutions. The final concentrations of shell monomers and BIS ranged from 85-94% and 1-10% by weight of a 40 mM total monomer solution, respectively. 5% Acrylic acid (AAc) was added before initiation with APS to facilitate chemoligation of the fibrin-binding antibody. Following synthesis, CS microgels were purified as described above. To create hollow microgels, CS microgels were incubated with 50 mM sodium periodate (NaIO₄) at room temperature for up to 48 hours to cleave the DHEBA crosslinks (diols).

**Microgel Characterization.** Microgel hydrodynamic diameters were determined in 1X HEPES (25 mM HEPES, 150 mM NaCl, pH 7.4) through DLS using a Zetasizer Nano S (Malvern Instruments, Worcestershire, UK). Diameters were averaged from 5 measurements (each measurement was averaged from 5 runs for 30 sec each). Microgel deformability was determined with AFM imaging (MFP-3D BIO AFM, Asylum Research, Santa Barbara, CA). Glass coverslips were cleaned by submerging in a series of solutions in an ultrasonic bath for 10 min each: alconox detergent, water, acetone, absolute ethanol, and isopropyl alcohol. Microgel suspensions were pipetted onto clean, dry coverslips and allow to dry overnight. AFM imaging was performed in tapping mode with silicon probes (ARROW-NCR, NanoAndMore, Watsonville, CA). Average diameter and heights +/- standard deviation were determined for at least 30 microgels per condition from at least 3 different images using ImageJ image analysis software (National Institutes of Health, Bethesda, MD). Microgel morphology was characterized in water using cryogenic scanning electron microscopy (CryoSEM) with a JEOL 7600F at 50000X magnification. At least 5 images were collected per condition. For a comparison to native platelets, blood was acquired from the New York Blood Center (New York City, NY) and platelets were isolated by centrifugation at 150 g for 15 min with no deceleration to remove red blood cells and the buffy coat. Additional centrifugation of the platelet-rich plasma at 900 g's for 5 minutes concentrated platelets into a pellet that was subsequently washed and resuspended in Tyrode's albumin buffer (Fisher Scientific, Hampton, NH). Platelets were activated by the addition of 0.25 U/mL human α-thrombin (Enzyme Research Laboratories, South Bend, IN) immediately before imaging.

**Analysis of fibrin clot collapse**. A fibrin-binding IgG antibody (Sheep anti-human Fibrin fragment E, Affinity Biologicals, Ancaster, ON) was conjugated to microgels through EDC/NHS coupling. Fibrin clots were prepared with a final fibrinogen concentration (FIB 3, Enzyme Research Laboratories, South Bend, IN) of 2 mg/mL, 10% of which was Alexa Fluor 488 conjugated fibrinogen (Fisher Scientific) to allow for visualization of the fibrin matrix. The clot was polymerized with 0.5 U/mL human α-thrombin (10% by volume) in the presence or absence of fibrin-binding or control microgels. The effect of microgels on microscopic clot structure was analyzed using fluorescence confocal microscopy (Zeiss LSM 710) on 25 µL clots, observed as overlaid z-stacks (63X water immersion objective, section step 0.63 µm). A minimum of 6 clots were analyzed for each treatment condition. Gross clot collapse over time of fibrin clots with and without CoreShell 2% BIS microgels treated with core degrading NaIO₄ was also analyzed through confocal microscopy. 25 µL fibrin clots with approximate mean cross sectional areas of 8.6 µm² were created in glass bottom dishes and imaged 1 and 48 hrs following polymerization. Clot area was measured as total cross sectional area from 10X tiled scans using ImageJ. Change in area over time was determined by comparing crosssectional areas at 48 hours to corresponding initial area. At least three clots were imaged per condition and average area ratios +/- standard deviations are presented. Statistical analysis was determined by a one-way ANOVA with a post hoc Tukey's multiple comparisons test with p < 0.05. A Shaprio-Wilk Normality test was run to check normality of data; these values passed the normality test. A Bartlett's test indicated that the variance of the groups did not differ significantly.

**Analysis of wound healing *in vivo.*** The effect of microgels on wound healing was assessed in an *in vivo* murine wound model of wound healing. All procedures were approved by the NCSU IACUC. Male C57/B6 mice were anesthetized using general anesthesia (5% isoflurane in 100% oxygen, flow rate 2 L/min, and maintained at 2-3% isoflurane) through a nose cone for the duration of surgery. A full thickness dermal wound extending through the subcutaneous tissue and panniculus carnosus was created and a topical application of microgels (10 µL of 2 mg/mL in 0.9% saline pH 7.4) was applied directly to the wound and control wounds received 0.9% saline pH 7.4 only. The wounds were splinted with 10 mm diameter silicone rings with 5 mm inner diameter holes and imaged. Microgel PLP treatment groups (saline, CoreShell 2% BIS, and Hollow 2% BIS) were applied topically to the site of the wound in a 10 µL solution at 4 mg/mL which is a relatively non-viscous solution, similar in consistency to saline, and not a "wound gel." The volume of 10 µL is small enough to remain on the 4 mm diameter wound site without rinsing off or leaving the wound border. Different treatments were applied topically to the 2 wounds created per mouse to diminish animal-specific variability with n=5 for each treatment, prior to suturing the silicone splint into place. Wounds were covered with a waterproof, but moisture vapor permeable Opsite wound dressing, applied to the surface of the silicone ring to cover, but not interact with the wounds. Before removing the mice from anesthesia, 5 mg/kg carprofen was administered via sub-cutaneous injection for post-operative pain relief and was then administered once daily for 5 days. Opsite wound dressings were removed for daily imaging and replaced with fresh dressings for 8 days. Images were analyzed to quantify wound closure as a measure of wound size compared to the inner area of the silicone splint normalized to each wound's initial area. The analysis of the wound size from daily images was blinded by treatment group and the order of analysis was randomized through a random integer generator. Wound boundaries were determined manually, primarily through visual inspection of apparent changes in color between the wound and healed areas. Apparent visual differences between tissue texture and height were also used to determine wound boundary.

Dermal tissue surrounding wounds was excised on day 8 following euthanasia, fixed, dehydrated, embedded in paraffin, and sectioned (10 µm). Sections were stained with Martius Scarlet Blue (MSB), and epidermal thickness was measured on MSB images at 5 regions evenly spaced throughout the wound area for 4 samples per group. Additional immunohistochemistry was performed on sister sections to visualize smooth muscle actin (α-SMA) and endothelial cell marker CD31 (PECAM-1), indicating the presence of newly formed blood vessels, or angiogenesis, which is a critical step in wound healing. For immunohistochemistry, tissue was deparaffinized, rehydrated, and antigen retrieval using citrate buffer was performed. Tissue was incubated with 5% bovine serum albumin (BSA, Fisher Scientific) in PBS prior to antibody labeling. Sections were co-labeled with polyclonal antibody to α-SMA (1:500, clone PA5-18292, Thermo Fisher Scientific, Waltham, MA) and monoclonal antibody to CD31 (1:50, clone SP38, Thermo Fisher Scientific, Waltham, MA) overnight at 4°C, washed, and labeled with secondary antibodies (Alexa 488 and Alexa 594, respectively) 45 minutes at room temperature. Sections were washed and mounted with Vectashield HardSet mounting medium with DAPI (Fisher Scientific), and imaged with fluorescence microscopy (EVOS FL Auto Cell Imaging System, Fisher Scientific). CD31 positively labeled tissue was quantified using ImageJ. CD31+ vessel number was obtained via 1) a manual count of fluorescent vessels in the red channel (presented in Figure 3) and 2) an automated method in which ImageJ Particle Analysis was to measure the number of vessels (using a size threshold of particles greater than 1.0 µm² and an intensity threshold of 0-50; shown in Figure 4) for at least 15 slides per treatment condition (n=25 for saline; n=15 for C/S; n=30 for hollow). For all quantitative analysis of healing parameters, rates of healing, epidermal thickness, and CD31+ vessel number, mean +/- SD are presented. A Shaprio-Wilk Normality test was run to check normality of data and a Bartlett's test was utilized to analyze variance of the groups. A Bartlett's test indicated that the variance of the groups did not differ significantly for epidermal thickness values, therefore an ANOVA with a post hoc Tukey's multiple comparisons test with significance determined as p<0.05 was used to analyze this data. Analysis of vessel counts indicated that these data did not pass the normality test and the variance of the groups differed significantly. Therefore, for the vessel count data, a Kruskal-Wallis with Dunn's multiple comparisons test was utilized to analyze statistical significance.

**Statistical analysis.** All statistical analyses were performed with Prism software program (GraphPad, San Diego CA). A Shaprio-Wilk Normality test was run to check normality of data and a Bartlett's test was utilized to analyze variance of the groups. When data passed normality tests and variances did not signficantly differ, data was analyzed using a one-way analysis of variance (ANOVA) with a Tukey's post hoc test at a 95% confidence interval. Data that did not pass these tests were analyzed using Kruskal-Wallis with Dunn's multiple comparisons test to analyze statistical significance. Means +/- standard deviation are reported. For all analyses, a minimum of three samples were analyzed per group. Any specific pre-processing of data (such as normalization) and specific sample sizes for each experimental are detailed in specific experimental sections above

### Example 2. Core-Shell Nanogels and Fibrinolytic Nanogels.

### Preparation of core-shell nanogels and fibrin-specific nanogels.

Core-shell (CS) poly(N-isopropylacrylamide) (pNIPAM) nanogels were synthesized through two precipitation polymerization reactions. In the first reaction, nanogel cores were synthesized using 90% N-isopropylacrylamide (NIPAM) (Sigma-Aldrich) monomer and 10% N,N'-methylenebisacrylamide (BIS) crosslinker (Sigma-Aldrich) at final concentrations of 140mM and dissolved in deionized water with 2mM sodium dodecyl sulfate (SDS) (Sigma-Aldrich). The solution was filtered with a 0.2µM filter and heated to 70°C in an oil bath while oxygen was purged from the system with nitrogen gas. The reaction was heated for 1 hour at 70°C and initiated with filtered ammonium persulfate (APS) (Sigma-Aldrich) at 2mM. The reaction was left to react under a blanket of nitrogen gas for 6 hours. Then, the solution was cooled to room temperature, filtered through glass wool and dialyzed against deionized water twice (24 hours each) and ultrapure water once (24 hours) using 1000kDa molecular weight cutoff dialysis tubing (Biotech CE Dialysis Tubing, Spectrum Laboratories). Studies with fluorescent particles incorporated 0.1% Methacryloxy ethyl thiocarbamoyl rhodamine B monomer (Polyscience) during core synthesis. For the second reaction, nanogel shells were synthesized around the cores using 93% NIPAM monomer and 2% BIS crosslinker at 40mM dissolved in deionized water with 0.5mM SDS. The solution was filtered with a 0.2µM filter and brought up to volume in a solution containing 40% by volume core solution synthesized from the first reaction. The solution was then heated to 70°C in an oil bath with nitrogen gas bubbling through. After 50 minutes at 70°C, 5% acrylic acid (AAc) was added, and the reaction was then initiated with a final concentration of 1mM APS 10 minutes later. The reaction was left to react under a blanket of nitrogen gas for 4 hours. Then, the solution was cooled to room temperature, filtered through glass wool, and dialyzed against deionized water twice (24 hours each) and ultrapure water once (24 hours) using 1000kDa MWCO dialysis tubing (Biotech CE Dialysis Tubing, Spectrum Laboratories). Fibrin-specific nanogels (FSNs) were created by conjugating a sheep anti-human fibrin fragment E polyclonal antibody (Affinity Biologicals) to C/S nanogels by utilizing the co-polymerized AAc functional handles in the nanogel shell and EDC/Sulfo-NHS (Thermo Scientific, Pierce) coupling. The FSNs were then dialyzed as mentioned previously and purified by lyophilizing.

### Characterization of core-shell microgels

Hydrodynamic diameters of core and C/S nanogels were determined using Nanosight particle tracking analysis (Malvern), which utilizes individual particle tracking analysis for particles moving under Brownian motion and the Stokes Einstein equation to calculate hydrodynamic diameters. For testing, nanogels were suspended in ultrapure water at 9×10⁸-2×10⁹ particles/mL and measured in 5 captures for 60 seconds each. AFM was conducted on glass coverslips. Coverslips were cleaned through 10-minute sonication periods in solutions of alconox, water, acetone, absolute ethanol, and then isopropyl alcohol. Then, coverslips were air dried and 10µL of a diluted nanogel suspension in ultrapure water was placed on the coverslip and allowed to dry overnight. Once completely dry, coverslips were adhered to glass microscope slides with cyanoacrylate and imaged on the AFM to determine particle height and diameter. At least 30 particles were measured for height and diameter for both cores and C/S nanogels.

### Characterization of fibrin specificity

*In vitro* binding studies of FSNs to characterize fibrin specificity were conducted through a microscopy based approach with a custom-made polydimethylsiloxane (PDMS) fluidics chamber that contained a fluorescent fibrin clot in the top reservoir reaching down to the T junction of the device. The fibrin clot was prepared with final concentrations of 2.5 mg/mL fibrinogen (FIB 3, Enzyme Research Laboratories), including 10% labeled fibrinogen, and 0.5U/mL human α-thrombin (Enzyme Research Laboratories). After polymerization of the fibrin clot for 1 hour, a solution of rhodamine B fluorescent FSNs was flowed through the channel at a WSR of 1sec⁻¹, a value similar to physiologic low WSR vessels. Baseline images were acquired immediately upon injection and images after 5, 10, 15, and 20 minutes of flow. Nanogel retention was imaged as well after 5, 10, 15, and 20 minutes of HEPES buffer wash (25mM HEPES, 150mM NaCl, 5mM CaCl₂ pH 7.4). Three trials were conducted per group.

### Evaluation of temporally controlled release kinetics of small and large molecules from core-shell nanogels

C/S nanogels were loaded with large and small dextran molecules (20kDa and 6kDa) (VWR) labeled with fluorescein isothiocyanate (FITC) and rhodamine B (RhoB) respectively. Loading was done in a one-step "breathing in" method in which lyophilized nanogels were rehydrated in loading solutions for 24 hours. Various ratios of the small and large dextran molecules were loaded into C/S nanogels, including 225µg/mL RhoB dextran (small alone), 225µg/mL FITC dextran (large alone), 225µg/mL RhoB dextran + 225µg/mL FITC dextran (1:1 small:large), 225µg/mL RhoB dextran + 112.5µg/mL FITC dextran (2:1 small:large), and 112.5µg/mL RhoB dextran + 225µg/mL FITC dextran (1:2 small:large). Following loading, particles were purified by centrifugation, washed with HEPES buffer, and centrifuged again. Nanogels were then put into HEPES solution, and at various time points (1, 2, 6, 18, 44, and 168 hours) the supernatant was collected. The release profiles were determined by analyzing fluorescence in the supernatant samples using a microplate reader (Biotek Synergy H1) (excitation-emission FITC: 490-525, RhoB: 540-625) and comparing to a standard curve of known fluorescent dextran concentrations. Three triplicate experiments were conducted.

### In vitro clot degradation of tPA and Y-27632-loaded core-shell particles

C/S nanogels were loaded through the "breathing in" method by swelling particles in the presence of a 414µM tPA (VWR) solution, a 414µM Y-27632 (Fisher Scientific) solution, or a solution containing both 414µM tPA and 414µM Y-27632 at particle concentrations of 20mg/mL. The activity and release kinetics of tPA from the nanogels was first evaluated by characterizing the fibrinolytic activity of 414µM tPA-loaded particle releasate overtime using an endogenous fibrinolysis assay. Releasate was collected following loading and 1x HEPES wash as particles were placed into HEPES buffer and at various time points (1, 2, 6, 18, 44, and 168 hours) the supernatant was collected. To test the tPA in released supernatant samples, clot lysis was evaluated using an absorbance-based endogenous fibrinolysis assay where 90µL clots were formed from 1mg/mL fibrinogen (Enzyme Research Laboratories), 0.25 U/mL human α-thrombin (Fisher Scientific), 10.8 µg/mL human plasminogen (Thermo Fisher Scientific) and 20% by volume sample supernatant. Clot absorbance at 350nm was monitored every 30 seconds using a plate reader. By conducting the endogenous fibrinolysis assay with known concentrations of tPA, a standard curve was generated based on to the time to reach maximum clot absorbance. Release samples were then fit to this standard curve to generate a cumulative release plot of tPA released from the C/S nanogels over time. Three triplicate experiments were conducted. Further testing the activity and release kinetics of tPA from single and dual-loaded nanogels, loaded particles were purified as mentioned previously and lyophilized. Three identical endogenous fibrinolysis assays were also performed in triplicate with *in vitro* fibrin clots directly in the presence of 2mg/mL single or dual-loaded C/S nanogels (instead of released supernatant).

### Live Dead Assay

Neonatal cardiac fibroblasts were cultured for 24 hours in the presence of 1mg/mL C/S nanogels. ReadyProbes Cell Viability Imaging Kit (Invitrogen) was used to access live and dead cells by staining for 15 minutes with both live and dead stains. Cells were imaged immediately after staining on an EVOS FL Auto Imaging System. Cell viability was determined as a percent of the fluorescence detected from the live or dead stains. Experiments were performed three times in triplicate.

### In vitro evaluation of fibrotic markers in response to tPA and Y-27632-loaded core-shell particles

Unless otherwise noted, DMEM media supplemented with 10% FBS, 1% L-glutamine, and 1% penicillin streptomycin was used to culture human dermal fibroblasts (HDFn) cells (Gibco). Neonatal rat cardiac fibroblasts (NRCF) were isolated as previously described. Unless otherwise noted, DMEM media supplemented with 10% FBS, 1% L-glutamine, 20mg/mL gentamycin, and 0.01mM 2-mercaptoethanol was used to culture NRCF cells. Passage 2-6 NRCF were used for all in vitro experiments.

Using single or dual-loaded C/S nanogels, the functionality of Y-27632 was first evaluated using HDFn as a model cell type. HDFn cells were cultured in serum free media on glass coverslips coated with 10µg/cm² collagen I (Enzo Life Sciences) and incubated with 1mg/mL particles. Cell circularity of the HDFn cells was monitored at time points of 6, 9, and 18 hours. Three experiments were conducted and 20-30 cells were measured for circularity at each time point for each experiment. Circularity was determined using the equation: circularity=4π(area/perimeter²). Experiments performed on NRCFs included culturing cells in serum free media on glass coverslips coated with 10µg/cm² collagen I and incubated in the presence of 1mg/mL of drug-loaded C/S nanogels for 24 hours. Cells were then fixed with 4% paraformaldehyde, permeabilized with 0.1% Triton-X 100, blocked with 1% BSA, and incubated with anti-actin, α-SMA, clone 1A4 (Sigma-Aldrich) or anti-CTGF antibody ab6992 (Abcam). AlexaFluor 488 goat anti-mouse (Invitrogen) and AlexaFluor 594 goat anti-rabbit (Invitrogen) were used as secondary antibodies for α-SMA and CTGF, respectively. Samples were mounted in Fluoromount-G mounting medium (Electron Microscopy Sciences) and images acquired with an EVOS FL Auto Imaging System (Invitrogen). Percent positive stress fibers were calculated in each experiment by analyzing a minimum of 5 images at 20x magnification per group. Cells containing distinct stress fibers were marked as positive. For each image, a percent of positive cells to total number of cells in the image was calculated. Quantification shows the averages of these percentages and three experiments were conducted. Expression levels were measured via corrected total cell fluorescence found by the following equation: Integrated density of cell-(cell area*mean gray value of background). Three experiments were conducted and 20-30 cells were measured for each experiment.

### In vivo studies of FSN retention at sites of MI

Female Sprague Dawley rats were induced with I/R injury. To induce I/R injury, rats underwent left thoracotomy under general anesthesia where MI was induced by ligation of the left anterior descending coronary artery. The coronary artery was ligated for 30 minutes. The sutures were then removed to permit reperfusion. Immediately following removal of the coronary artery ligation, rats received treatment injections in the ventricular cavity.

To determine if FSNs would be retained at sites of MI *in vivo,* fluorescent FSNs were injected into female Sprague Dawley rats after inducing I/R injury. Following removal of the suture, rats received injections of either PBS, non-binding nanogels (50uL of 1mg/mL solution), or binding FSNs (50uL of 1mg/mL solution). Samples were directly injected into the ventricular cavity following reperfusion. Animals were sacrificed after 24 hours and harvested organs were fluorescently imaged *ex vivo* with an IVIS Xenogen *In Vivo* Imager. 5 rats were analyzed per group. Quantification was done as a percent change in fluorescence intensity compared to PBS controls.

### In vivo studies of functional outcome following MI and dual-loaded FSN treatment

For *in vivo* studies to assess functional outcome following I/R injury, FSNs were dual-loaded with 414µM Y-27632 and/or 414µM tPA using the "breathing in" method. These experiments utilized the I/R rodent model in female Sprague Dawley rats. Following I/R injury, rats received 50µL injections of PBS, non-binding non-loaded C/S nanogels, binding non-loaded C/S nanogels, binding tPA-loaded C/S nanogels, binding Y-27632-loaded C/S nanogels, or binding dual-loaded C/S nanogels. Samples were directly injected into the ventricular cavity following reperfusion. Left ventricular ejection fraction and fractional shortening was measured via echocardiography 4 hours, 2 weeks, and 4 weeks post-surgery. Rats were sacrificed at 4 weeks and harvested organs were frozen in OCT compound. Specimens were cryosectioned (Thermo Scientific HM525 NX) every 200µm (10µm thickness). Masson's trichrome staining was performed and from stained images morphometric parameters were measured, including scar size as a percent of left ventricular area. Immunohistochemistry was performed on the 4-week, 4% paraformaldehyde-fixed histological sections at infarct sites. Primary antibodies included α-SMA and CTGF, which are markers of myofibroblast differentiation to evaluate fibrosis associated responses. 5 rats were analyzed/group.

### Statistical analysis

Statistical analyses for *in vitro* data were performed with Prism software program (GraphPad). Data was analyzed using a one-way analysis of variance (ANOVA) using a Tukey multiple comparisons test at a 95% confidence interval. For *in vivo* functional outcome data, a two-way analysis of variance (ANOVA) was conducted with a Tukey multiple comparisons test at a 95% confidence interval.

### Characterization of core-shell nanogels

In order to generate our therapeutic nanogels, we first synthesized core-shell (C/S) particles using 10% of N,N'-methylenebisacrylamide (BIS) crosslinker in the core and 2% BIS crosslinker in the shell. These crosslinking densities were chosen to control the uptake and release of two distinct therapeutic molecules within the particle layers. Dry diameter and height measurements from atomic force microscopy (AFM) analysis demonstrates that core nanogels imaged on a glass surface are 181 nm in diameter and 20 nm in height. After shell addition, the diameter increases to 238 nm and particle height increases to 29 nm (Figure 6, panels B, D, and E). Additionally, hydrodynamic dimensions from Nanosight analysis shows a core diameter of 162 nm and C/S nanogel diameter of 244 nm (Figure 6, panels B and C). These data validate successful shell addition to core particles and demonstrate that C/S nanogels can be successfully created using our synthesis method to fabricate C/S nanogels with distinct core and shell crosslinking densities.

### Evaluation of temporally controlled release kinetics of small and large molecules from core-shell nanogels

Following particle synthesis and characterization, we examined the ability of a core-shell particle design with differential crosslinking densities to independently vary uptake and release rates from the core and the shell. Using large (20kDa) FITC and small (6kDa) Rhodamine B labeled dextran molecules and comparing various loading ratios of the small and large dextran molecules into C/S nanogels, it was found that a 2:1 small:large ratio allows for the most extended release of small molecules (Figure 7). All loading conditions show essentially complete release of the larger 20kDa dextran within 6 hours. Comparatively, 2:1 small:large loading solutions showed an average release of 19µg/mL of smaller 6kDa molecules at 6 hours and remained at detectable levels up to 168 hours. Collectively, these data suggest that our C/S design allows for temporally controlled release kinetics of small and large molecules in a single delivery platform that can independently vary release rates from the individual layers.

### In vitro clot degradation of tPA and Y-27632-loaded core-shell particles

Following experiments with model dextran molecules, functional uptake and release of tPA into the C/S nanogels was examined. Following loading of C/S nanogels through a rehydration technique, releasate samples were tested for their ability to degrade fibrin clots. All releasate samples from tPA-loaded C/S nanogels resulted in clot degradation *in vitro,* seen with decreases in absorbance values (higher absorbance corresponds to polymerized clots while decreases in absorbance corresponds to clot degradation). These data demonstrate functional release of tPA from the particles. The cumulative release plots generated show a large burst release response of tPA followed by small amounts of release up to 168 hours (Figure 8, panel A). Analysis from three triplicate experiments to the standard curve of known tPA concentrations indicates an average concentration of 0.37 +/- 0.06 µg of tPA released per mg of loaded C/S nanogels. Directly incorporating tPA-loaded C/S nanogels (with or without Y-27632 present) into polymerizing clots rapidly degrades clots *in vitro,* indicating that our design facilitates a burst release of functional tPA from the tPA-loaded nanogels (Figure 8, panel B).

### Core-shell particles do not affect cellular viability

To determine potential toxicity of C/S nanogels on cardiac fibroblasts, a live:dead analysis of neonatal rat cardiac fibroblasts cultured in the presence of unloaded particles was conducted. These studies demonstrate that cardiac cells maintain a high level of viability in the presence of unloaded C/S nanogels comprised of a 2% BIS shell and a 10% BIS core.

### In vitro evaluation of fibrotic markers in response to tPA and Y-27632-loaded core-shell particles

Following experiments to determine functionality of tPA loading and release, functional uptake and release of Y-27632 into the C/S nanogels was examined. After loading of C/S nanogels through a rehydration technique, expression of fibrotic markers was examined in neonatal rat cardiac fibroblasts. Cardiac fibroblasts cultured in the presence of Y-27632-loaded C/S nanogels (with or without tPA present) for 24 hours showed a reduced percent of α-smooth muscle antibody (α-SMA) positive stress fiber forming cells and a downregulation of CTGF compared to control fibroblasts (Figure 9). α-SMA and CTGF were examined as they are known to be upregulated in fibrotic responses. Similar results in α-SMA expression were also seen in HDFn cells. In the presence of dual-loaded nanogels, cells exhibited a downregulation of α-SMA, comparable to the response of cells in the direct presence of 10µM Y-27632. Increasing the concentration of Y-27632 in the dual-loading solution from 50µM to 414µM further enhanced this response. Cellular morphological changes in HDFn cells also demonstrated functional ROCK inhibitor release as fibroblast cells exhibited a more circular morphology and less contractility in the presence of 414µM Y-27632 + 414µM tPA loaded nanogels and elongated fibroblasts are characteristic of increased contractility. Collectively, these data demonstrate that Y-27632-loaded C/S nanogels release functional ROCK inhibitor to decrease cellular contractility, reduce stress fiber formation, and decrease CTGF expression.

### Fibrin specificity of core-shell nanogels in vitro and in vivo

After demonstrating the functional loading and release of tPA and Y-27632 in C/S nanogels, the fibrin binding of these particles was examined. C/S nanogels were coupled to a fibrin-specific antibody to form fibrin-specific C/S nanogels (FSNs). Under flow conditions *in vitro,* FSNs demonstrated binding capabilities and retention to fibrin clots at physiologically similar wall shear rates (WSR) of 1sec⁻¹ (Figure 10). Non-binding C/S nanogels exhibit no particle accumulation at clot boundary sites at these flow conditions. Additionally, FSNs and non-binding nanogels were directly injected into the ventricular cavity of an I/R rodent model. After 24 hours, fluorescent detection compared to PBS controls showed FSNs localizing to damaged heart tissue with some accumulation in the kidney, but limited accumulation in the lungs or liver (Figure 11). Conversely, non-binding nanogels were found to accumulate more in the lung, liver, and kidney rather than the damaged heart tissue when comparing fluorescent levels to PBS controls.

### Dual-loaded FSNs enhance functional outcomes following MI

With FSNs showing *in vitro* and *in vivo* localization to sites of fibrin clots and damaged heart tissue, we examined the functional outcomes of drug-loaded FSNs in an I/R rodent model. Following reperfusion and injection of the treatment groups, echocardiography analysis shows that the treatment groups show similar acute infarct cardiac malfunction in regard to left ventricular ejection fractions 4 hours post-I/R (Figure 12, panel A). Results demonstrate that at 2 weeks post-I/R dual loaded FSNs significantly enhance EF compared to all other groups. This improvement is sustained 4 weeks post-I/R as well. Some EF improvements are seen with Y-27632 only loaded nanogels at 2 weeks. However, this improvement is not sustained at 4 weeks. tPA only loaded nanogels have a slightly higher baseline EF, although limited improvements in EF are seen 2 and 4 weeks post-I/R. Significant improvements in fractional shortening were also seen with dual-loaded FSNs 2 and 4 weeks post-I/R as well. Morphometric analysis shows scar size measurements at 4 weeks post-I/R (Figure 12, panels B and C). Larger infarct sizes are seen in the control groups and single drug-loaded treatment groups compared to the dual-loaded FSN group.

Immunohistochemistry analysis show both α-SMA and CTGF expression are downregulated in the dual-loaded, binding nanogel treatment group 4 weeks post-MI and I/R compared to control groups as well as single drug-loaded groups, suggesting higher capabilities of dual-loaded FSNs inhibiting cardiac fibrosis following reperfusion injury (Figure 12, panel D).

### Conclusions

In conclusion, we have found that C/S nanogels can be synthesized to individually control the loading and release of two distinct molecules. C/S nanogels can be loaded with fibrinolytic protein tPA as well as ROCK inhibitor Y-27632 and can degrade fibrin clots as well as downregulate fibrotic markers α-SMA and CTGF in cardiac fibroblasts *in vitro.* Particles with fibrin-specificity exhibit accumulation to damaged heart tissue *in vivo,* and loading these particles with tPA and Y-27632 shows enhanced cardiac function in an I/R rodent model, seen through improved ejection fraction, decreased infarct size, and decreased fibrotic response. Single drug-delivery of Y-27632 is not enough to improve cardiac function, significantly reduce scar size, or decrease fibrotic responses, perhaps due to high amounts of fibrin deposited at MI diseased sites. Additionally, tPA delivery alone is not enough to improve cardiac function or significantly reduce scar size perhaps because it does not combat the fibrotic response to reperfusion injury in which cell contractility plays a critical role. Therefore, by combining the efforts of tPA to reduce the fibrin accumulation and deposition at sites of MI with Y-27632 to mitigate cell contractility and cardiac fibrosis following reperfusion injury, it is possible to then see improvements in ejection fraction, scar size, and fibrotic markers.

A potential limitation of this approach is the ability of FSNs to remain at sites of MI following dissolution of the clot due to tPA delivery. However, results suggest that particles are retained at the injury site long enough to deliver sufficient amounts of Y-27632 to inhibit fibrosis. Additionally, significant improvements are still seen regarding cardiac function 4 weeks post-I/R, suggesting Y-27632 action to inhibit fibrosis which is not seen in non-Y-27632-loaded treatment groups. Another potential pitfall is particle retention only to the outside of tissue. It does not appear that particles penetrate the heart tissue. However, as previously suggested, this does not appear to limit the ability to deliver sufficient amounts of therapeutics that can improve cardiac function.

Here, we have shown a dual-delivery microgel strategy to treat acute and chronic myocardial infarction by controlling the release of fibrinolytic protein tPA and cell contractility inhibitor Y-27632 from a core-shell nanogel design that localizes the release of these therapeutics to sites of injury through nanogel fibrin-specificity. This design addresses current limitations in therapies for both reperfusion and reperfusion-injury associated fibrosis and has demonstrated its ability to be used as an MI therapeutic strategy that can 1) rapidly reestablish blood flow by degrading clot blockages and 2) inhibit cardiac fibrosis following reperfusion injury.

### Synthesis of monophase TPA-loaded nanogel

Poly(N-isopropylacrylamide) (pNIPAM) nanogels were synthesized in a precipitation polymerization reaction. The nanogel core synthesis incorporates 90% NIPAM and 10% methylenebisacrylamide (BIS) crosslinker. The nanogel shell includes 93% NIPAM, 2% BIS crosslinker, and 5% acrylic acid as chemoligation sites for EDC/NHS coupling of a fibrin-specific antibody. Nanogel hydrodynamic diameter was found through Nanosight particle tracking and dry particle diameter/height were determined through atomic force microscopy (AFM). Particles were purified via dialysis, lyophilized, and then rehydrated at 20 mg/mL for 24 hours with a 29 µg/mL tPA loading solution. Following loading, particles were purified via centrifugation and lyophilized. FSN fibrin binding was examined using a custom polydimethylsiloxane (PDMS) fluidic device (Figure 17, panels A and B).

Clotting characteristics were evaluated using an absorbance-based polymerization/degradation assay (Figure 5, panel C) where 90uL clots were formed from 1 mg/mL fibrinogen, 0.25 U/mL thrombin, 10.8 µg/mL plasminogen, and 2 mg/mL nanogels (FSNs or tPA-FSNs) in HEPES buffer (25 mM HEPES, 150 mM NaCl, 5 mM CaCl2). Clot absorbance at 350 nm was monitored every 30 seconds on a plate reader. Clot polymerization/degradation was also analyzed using the device in Figure 17, panel A. Here, fibrin clots (2.5 mg/mL fibrinogen, 0.5 U/mL thrombin in buffer) were formed using 10% fluorescently labeled fibrinogen to allow for visualization. Fibrin clots were polymerized for 1 hour. Then, solutions of 2.5 mg/mL fibrinogen, 0.1 U/mL thrombin, and 10.8 ug/mL plasminogen in buffer with or without 1 mg/mL nanogels (FSNs or tPA-FSNs) were flowed through the device at wall shear rates of 1sec⁻¹ using a syringe pump. Images at the clot boundary were taken every minute for 20 minutes. Endpoint images of upstream clotting were also examined (Figure 17, panel D).

The hydrodynamic diameter of pNIPAM particles was 244 ± 36 nm. AFM analysis demonstrates a particle dry diameter of 238 ± 30 nm and height of 29 ± 6 nm. FSNs exhibit fibrin-binding at wall shear rates of 1 sec-1 (Figure 17, panel B). Dynamic polymerization/fibrinolysis assays demonstrate that FSNs alone do not facilitate fibrin clot lysis. However, tPA-FSNs show degradation characteristics and clot destabilization (Figure 17, panel C). Under flow, clotting characteristics seen in upstream endpoint images illustrate significant clot augmentation in the presence of FSNs. In the presence of tPA-FSNs, enhanced clotting is seen at the stationary boundary conditions, but does not overtake the entire channel (Figure 17, panel D).

### Tissue plasminogen activator (tPA)-loaded FSNs (core-shell microgels conjugated to fibrin-specific antibody) for the treatment of DIC

Tissue plasminogen activator (tPA)-loaded FSNs (core-shell microgels conjugated to fibrin-specific antibody) have been studied for treatment of the thrombotic disorder, disseminated intravascular coagulation (DIC). To examine clotting characteristics with tPA-FSNs in conditions of active coagulation *in vitro,* a custom-made polydimethylsiloxane fluidics chamber was utilized, allowing for a fibrinogen and thrombin containing solution to flow through a channel in the presence of a pre-polymerized fibrin clot at a T-intersection. In the presence of low thrombin levels, some clot augmentation occurs (Figure 18). Upon the addition of FSNs, significantly more clot augmentation occurs as particles aid in cross-linking fibrin fibers under circumstances of active coagulation. However, if no thrombin is present, FSN particles do not augment clot growth. Additionally, in the presence of microgels without conjugated fibrin-specific antibody, clot growth is similar to control conditions with only fibrinogen and thrombin. In the presence of tPA-loaded FSNs, particles bind to clot sites at the T-intersection and clot degradation occurs (Figure 18).

To examine tPA-FSNs capabilities in treating DIC *in vivo,* a lipopolysaccharide-induced rodent model of (DIC) was used where DIC was induced by an infusion of lipopolysaccharide (30mg/kg) for 4 hours. Then, animals were treated with either saline, unloaded FSNs, or tPA-loaded FSNs (tPA-FSN). Blood samples were taken and organs were harvested after 30 minutes of treatment and compared to naive wildtype animals. Fibrinogen, D-dimer, platelet counts, and organ weights were examined. This data shows reduced fibrinogen and elevated D-dimer levels in all DIC animals demonstrating microthrombi formation and high fibrin turnover in this animal model, a common complication seen in DIC. Platelet count demonstrates recovery to normal, wildtype levels after tPA-FSN treatment (Figure 19).

In DIC-induced animals, whole organ images and histological sections show darkened organ color and hemorrhagic areas as well as microthrombi presentation, which are diminished in tPA-FSN treatment group (Figure 20). Fibrin deposition is quantified using immunohistochemistry for fibrin and ImageJ particle analysis and shows significantly less fibrin deposition in tPA-FSN treatment group compared to other DIC animals (Figure 20). Clot structure was evaluated in plasma samples using CryoSEM and confocal microscopy. Clots were formed with thrombin and, for confocal microscopy, with Alexa-488 fibrinogen for fiber visualization. Clot porosity and fiber density were evaluated using ImageJ. Limited fiber formation signifies consumption of clotting factors *in vivo* in DIC compared to wildtype animals. Improved clot structure formation after tPA-FSN treatment signifies recovery of some clotting factor consumption (Figure 21).

## Claims

1. A fibrinolytic nanogel, comprising: a crosslinked polymer network, fibrin binding moiety and tissue plasminogen activator, wherein the fibrinolytic nanogel is hollow particles.

2. A fibrinolytic nanogel according to claim 1, wherein the hollow particles comprise a shell formed from a polymeric matrix enveloping a hollow core.

3. The fibrinolytic nanogel of claim 1 or claim 2, wherein the crosslinked polymer network is derived from a monofunctional monomer comprising an acrylamide, a (meth)acrylic acid, a vinyl alcohol, a polyalcohol, a vinyl pyrrolidone, or a copolymer thereof, and a crosslinking monomer, preferably wherein the monofunctional monomer comprises a combination of an acrylamide and a (meth)acrylic acid, and preferably wherein the crosslinking monomer comprises a polyfunctional acrylate, polyfunctional acrylamide, or combination thereof.

4. The fibrinolytic nanogel of claim 3, wherein the monofunctional monomer comprises a combination of N-isopropylacrylamide and acrylic acid.

5. The fibrinolytic nanogel of any of claims 1-4, wherein the fibrin binding moiety is conjugated to (meth)acrylic acid residues in the crosslinked polymer network.

6. A platelet-like particle, comprising a shell enveloping a hollow core, wherein the shell comprises a crosslinked polymer network conjugated to a fibrin binding moiety.

7. The platelet-like particle of claim 6, wherein the crosslinked polymer network is derived from a mixture of monomers, wherein the mixture comprises an acrylamide, a (meth)acrylic acid, a vinyl alcohol, an alkylene glycol, a vinyl pyrrolidone, or a combination thereof, and a crosslinking monomer, preferably wherein the mixture comprises an acrylamide or a combination of an acrylamide and a (meth)acrylic acid, and preferably wherein the crosslinking monomer comprises a polyfunctional acrylate, polyfunctional acrylamide, or combination thereof.

8. The platelet-like particle of claim 7, wherein the mixture comprises a combination of N-isopropylacrylamide and acrylic acid.

9. The platelet-like particle of any of claims 6-8, further comprising one or more therapeutic agents, preferably wherein the therapeutic agent comprises a fibrinolytic agent, preferably wherein the fibrinolytic agent is tissue plasminogen activator ("tPA").

10. A method for preparing the platelet-like particle of any of claims 6-9, comprising (a) providing a degradable core; (b) polymerizing a mixture of the monofunctional monomer and crosslinking monomer to form a shell around the core, (c) degrading the degradable core; and (d) conjugating the resulting nanogel with a fibrin binding moiety.

11. A composition comprising the platelet-like particles of any of claims 6-9 for use in a method of promoting wound healing or treating a thrombotic event or coagulative disorder.

12. A core-shell nanogel, comprising:
a core comprising a first crosslinked polymer network; and
a shell comprising a second crosslinked polymer network conjugated to a fibrin binding moiety.

13. The core-shell nanogel of claim 12, wherein the first crosslinked polymer network is derived from a first monofunctional monomer comprising an acrylamide, a (meth)acrylic acid, a vinyl alcohol, an alkylene glycol, a vinyl pyrrolidone, or a combination thereof, and a first crosslinking monomer, preferably wherein the first crosslinking monomer comprises an acrylamide; and/or wherein the first crosslinking monomer comprises a polyfunctional acrylate, polyfunctional acrylamide, or combination thereof;
wherein the second crosslinked polymer network is derived from a second monofunctional monomer comprising an acrylamide, a (meth)acrylic acid, a vinyl alcohol, a polyalcohol, a vinyl pyrrolidone, or a copolymer thereof, and a second crosslinking monomer, preferably wherein the second monomer comprises a combination of an acrylamide and a (meth)acrylic acid.

14. The core-shell nanogel of any of claims 12-13, wherein the second monomer comprises a combination of N-isopropylacrylamide and acrylic acid.

15. The core-shell nanogel of any claims 12-14, comprising at least one small molecule therapeutic agent, preferably wherein the shell further comprises a tissue plasminogen activator.

16. A method for preparing the core-shell nanogel of any of claims 12-15, comprising polymerizing a mixture of the first monofunctional monomer and first crosslinking monomer, separating the resulting particles from unreacted monomer, polymerizing a mixture of said particles, second monofunctional monomer, and second crosslinking monomer, and conjugating the resulting nanogel with a fibrin binding moiety.

## Patentansprüche

1. Fibrinolytisches Nanogel, umfassend: ein vernetztes Polymernetzwerk, eine Fibrinbindungseinheit und einen Gewebeplasminogenaktivator, wobei es sich bei dem fibrinolytischen Nanogel um hohle Teilchen handelt.

2. Fibrinolytisches Nanogel nach Anspruch 1, wobei die hohlen Teilchen eine Hülle umfassen, die aus einer Polymermatrix gebildet ist, die einen hohlen Kern umhüllt.

3. Fibrinolytisches Nanogel nach Anspruch 1 oder Anspruch 2, wobei das vernetzte Polymernetzwerk von einem monofunktionellen Monomer abgeleitet ist, das ein Acrylamid, eine (Meth)acrylsäure, einen Vinylalkohol, einen Polyalkohol, ein Vinylpyrrolidon oder ein Copolymer davon und ein vernetzendes Monomer umfasst, wobei das monofunktionelle Monomer vorzugsweise eine Kombination eines Acrylamids und einer (Meth)acrylsäure umfasst und wobei das vernetzende Monomer vorzugsweise ein polyfunktionelles Acrylat, polyfunktionelles Acrylamid oder eine Kombination davon umfasst.

4. Fibrinolytisches Nanogel nach Anspruch 3, wobei das monofunktionelle Monomer eine Kombination von N-Isopropylacrylamid und Acrylsäure umfasst.

5. Fibrinolytisches Nanogel nach einem der Ansprüche 1 bis 4, wobei die Fibrinbindungseinheit an (Meth)acrylsäurereste im vernetzten Polymernetzwerk konjugiert ist.

6. Plättchenartiges Teilchen, das eine Hülle umfasst, die einen hohlen Kern umhüllt, wobei die Hülle ein vernetztes Polymernetzwerk umfasst, das an eine Fibrinbindungseinheit konjugiert ist.

7. Plättchenartiges Teilchen nach Anspruch 6, wobei das vernetzte Polymernetzwerk von einer Mischung von Monomeren abgeleitet ist, wobei die Mischung ein Acrylamid, eine (Meth)acrylsäure, einen Vinylalkohol, ein Alkylenglykol, ein Vinylpyrrolidon oder eine Kombination davon und ein vernetzendes Monomer umfasst, wobei die Mischung vorzugsweise ein Acrylamid oder eine Kombination eines Acrylamids und einer (Meth)acrylsäure umfasst und wobei das vernetzende Monomer vorzugsweise ein polyfunktionelles Acrylat, polyfunktionelles Acrylamid oder eine Kombination davon umfasst.

8. Plättchenartiges Teilchen nach Anspruch 7, wobei die Mischung eine Kombination von N-Isopropylacrylamid und Acrylsäure umfasst.

9. Plättchenartiges Teilchen nach einem der Ansprüche 6 bis 8, ferner umfassend ein oder mehrere therapeutische Mittel, wobei das therapeutische Mittel vorzugsweise ein fibrinolytisches Mittel umfasst, wobei das fibrinolytische Mittel vorzugsweise Gewebeplasminogenaktivator ("tPA") ist.

10. Verfahren zur Herstellung des plättchenartigen Teilchens nach einem der Ansprüche 6 bis 9, umfassend (a) Bereitstellen eines abbaubaren Kerns; (b) Polymerisieren einer Mischung aus dem monofunktionellen Monomer und dem vernetzenden Monomer, um eine Hülle um den Kern herum zu bilden, (c) Abbauen des abbaubaren Kerns; und (d) Konjugieren des resultierenden Nanogels mit einer Fibrinbindungseinheit.

11. Zusammensetzung, die die plättchenartigen Teilchen nach einem der Ansprüche 6 bis 9 umfasst, zur Verwendung in einem Verfahren zur Förderung der Wundheilung oder zur Behandlung eines thrombotischen Ereignisses oder einer Gerinnungsstörung.

12. Kern-Hülle-Nanogel, umfassend:
einen Kern, der ein erstes vernetztes Polymernetzwerk umfasst; und
eine Hülle, die ein zweites vernetztes Polymernetzwerk umfasst, das an eine Fibrinbindungseinheit konjugiert ist.

13. Kern-Hülle-Nanogel nach Anspruch 12, wobei das erste vernetzte Polymernetzwerk von einem ersten monofunktionellen Monomer abgeleitet ist, das ein Acrylamid, eine (Meth)acrylsäure, einen Vinylalkohol, ein Alkylenglykol, ein Vinylpyrrolidon oder eine Kombination davon und ein erstes vernetzendes Monomer umfasst, wobei das erste vernetzende Monomer vorzugsweise ein Acrylamid umfasst; und/oder wobei das erste vernetzende Monomer ein polyfunktionelles Acrylat, polyfunktionelles Acrylamid oder eine Kombination davon umfasst,
wobei das zweite vernetzte Polymernetzwerk von einem zweiten monofunktionellen Monomer abgeleitet ist, das ein Acrylamid, eine (Meth)acrylsäure, einen Vinylalkohol, einen Polyalkohol, ein Vinylpyrrolidon oder ein Copolymer davon und ein zweites vernetzendes Monomer umfasst, wobei das zweite Monomer vorzugsweise eine Kombination eines Acrylamids und einer (Meth)acrylsäure umfasst.

14. Kern-Hülle-Nanogel nach einem der Ansprüche 12 bis 13, wobei das zweite Monomer eine Kombination von N-Isopropylacrylamid und Acrylsäure umfasst.

15. Kern-Hülle-Nanogel nach einem der Ansprüche 12 bis 14, umfassend mindestens ein kleines Molekül, ein therapeutisches Mittel, wobei die Hülle vorzugsweise ferner einen Gewebeplasminogenaktivator umfasst.

16. Verfahren zur Herstellung des Kern-Hülle-Nanogels nach einem der Ansprüche 12 bis 15, umfassend das Polymerisieren einer Mischung aus dem ersten monofunktionellen Monomer und dem ersten vernetzenden Monomer, Trennen der resultierenden Teilchen von nicht umgesetztem Monomer, Polymerisieren einer Mischung der Teilchen, des zweiten monofunktionellen Monomers und des zweiten vernetzenden Monomers und Konjugieren des resultierenden Nanogels mit einer Fibrinbindungseinheit.

## Revendications

1. Nanogel fibrinolytique, comprenant : un réseau de polymères réticulés, une fraction de liaison à la fibrine et un activateur tissulaire du plasminogène, dans lequel le Nanogel fibrinolytique consiste en des particules creuses.

2. Nanogel fibrinolytique selon la revendication 1, dans lequel les particules creuses comprennent une coquille formée d'une matrice polymère enveloppant un coeur creux.

3. Nanogel fibrinolytique selon la revendication 1 ou la revendication 2, dans lequel le réseau de polymères réticulés est dérivé d'un monomère monofonctionnel comprenant un acrylamide, un acide (méth)acrylique, un alcool vinylique, un polyalcool, une pyrrolidone vinylique, ou un copolymère de ceux-ci, et d'un monomère réticulant, de préférence dans lequel le monomère monofonctionnel comprend une combinaison d'un acrylamide et d'un acide (méth)acrylique, et de préférence dans lequel le monomère réticulant comprend un acrylate polyfonctionnel, un acrylamide polyfonctionnel, ou une combinaison de ceux-ci.

4. Nanogel fibrinolytique selon la revendication 3, dans lequel le monomère monofonctionnel comprend une combinaison de N-isopropylacrylamide et d'acide acrylique.

5. Nanogel fibrinolytique selon l'une des revendications 1 à 4, dans lequel la fraction de liaison à la fibrine est conjuguée à des résidus d'acide (méth)acrylique dans le réseau de polymères réticulés.

6. Particule de type plaquettaire, comprenant une coquille enveloppant un coeur creux, dans lequel la coquille comprend un réseau de polymères réticulés conjugué à une fraction de liaison à la fibrine.

7. Particule de type plaquettaire selon la revendication 6, dans lequel le réseau de polymères réticulés est dérivé d'un mélange de monomères, dans lequel le mélange comprend un acrylamide, un acide (méth)acrylique, un alcool vinylique, un alkylène glycol, une pyrrolidone vinylique, ou une combinaison de ceux-ci, et un monomère réticulant, de préférence dans lequel le mélange comprend un acrylamide ou une combinaison d'un acrylamide et d'un acide (méth)acrylique, et de préférence dans lequel le monomère réticulant comprend un acrylate polyfonctionnel, acrylamide polyfonctionnel, ou une combinaison de ceux-ci.

8. Particule de type plaquettaire selon la revendication 7, dans lequel le mélange comprend une combinaison de N-isopropylacrylamide et d'acide acrylique.

9. Particule de type plaquettaire selon l'une des revendications 6 à 8, comprenant en outre un ou plusieurs agents thérapeutiques, de préférence dans lequel l'agent thérapeutique comprend un agent fibrinolytique, de préférence dans lequel l'agent fibrinolytique est un activateur tissulaire du plasminogène (« tPA »).

10. Procédé pour préparer la particule de type plaquettaire selon l'une des revendications 6 à 9, comprenant (a) la fourniture d'un coeur dégradable ; (b) la polymérisation d'un mélange du monomère monofonctionnel et d'un monomère réticulant pour former une coquille autour du coeur, (c) la dégradation du coeur dégradable ; et (d) la conjugaison du Nanogel résultant avec une fraction de liaison à la fibrine.

11. Composition comprenant les particules de type plaquettaire selon l'une des revendications 6 à 9, pour son utilisation dans un procédé de promotion de la cicatrisation des plaies ou de traitement d'un événement thrombotique ou d'un trouble de la coagulation.

12. Nanogel coeur-coquille, comprenant :
un coeur comprenant un premier réseau de polymères réticulés ; et
une coquille comprenant un deuxième réseau de polymères réticulés conjugués à une fraction de liaison à la fibrine.

13. Nanogel coeur-coquille selon la revendication 12, dans lequel le premier réseau de polymères réticulés est dérivé d'un premier monomère monofonctionnel comprenant un acrylamide, un acide (méth)acrylique, un alcool vinylique, un alkylène glycol, une pyrrolidone vinylique, ou une combinaison de ceux-ci, et d'un premier monomère réticulant, de préférence dans lequel le premier monomère réticulant comprend un acrylamide ; et/ou dans lequel le premier monomère réticulant comprend un acrylate polyfonctionnel, un acrylamide polyfonctionnel, ou une combinaison de ceux-ci ;
dans lequel le deuxième réseau de polymères réticulés est dérivé d'un deuxième monomère monofonctionnel comprenant un acrylamide, un acide (méth)acrylique, un alcool vinylique, un polyalcool, une pyrrolidone vinylique, ou un copolymère de ceux-ci, et d'un deuxième monomère réticulant, de préférence dans lequel le deuxième monomère comprend une combinaison d'un acrylamide et d'un acide (méth)acrylique.

14. Nanogel coeur-coquille selon l'une des revendications 12 et 13, dans lequel le deuxième monomère comprend une combinaison de N-isopropylacrylamide et d'acide acrylique.

15. Nanogel coeur-coquille selon l'une des revendications 12 à 14, comprenant au moins un agent thérapeutique à petite molécule, de préférence dans lequel la coquille comprend en outre un activateur tissulaire du plasminogène.

16. Procédé de préparation du Nanogel coeur-coquille selon l'une des revendications 12 à 15, comprenant la polymérisation d'un mélange du premier monomère monofonctionnel et d'un premier monomère réticulant, la séparation des particules résultantes du monomère n'ayant pas réagi, la polymérisation d'un mélange desdites particules, d'un deuxième monomère monofonctionnel et d'un deuxième monomère réticulant, et la conjugaison du Nanogel résultant avec une fraction de liaison à la fibrine.
